# EUROPEAN PATENT SPECIFICATION

(11) **EP 0 479 245 B1**
(45) Date of publication and mention of the grant of the patent: **05.03.1997**
(21) Application number: 91116779.9
(22) Date of filing: 01.10.1991
(51) Int. Cl.: C08F 246/00, C08F 220/34, A61L 15/00, C08F 220/60, C08F 251/00, C08F 255/02

(54) **Absorbent polymer**
Absorbierendes Polymer
Polymère absorbant

(30) Priority: 01.10.1990 US 591301; 12.10.1990 US 596180; 31.10.1990 US 607005; 20.12.1990 US 632226; 11.02.1991 US 653581; 07.03.1991 US 665880
(43) Date of publication of application: 08.04.1992
(73) Proprietor: PHILLIPS PETROLEUM COMPANY, Bartlesville Oklahoma 74004 (US)
(72) Inventor: Ahmed, Igbal, Bartlesville, OK 74006 (US); Hsieh, Henry Lien, Bartlesville, OK 74006 (US)
(74) Representative: Geissler, Bernhard, Dr.jur., Dipl.-Phys.

(56) References cited:
- EP-A- 0 068 189
- EP-A- 0 191 980
- FR-A- 2 582 663
- GB-A- 1 524 899
- CHEMICAL ABSTRACTS, vol. 114, no. 6, 11 February 1991, Columbus, Ohio, US; abstract no 44600

## Description

This invention pertains to superabsorbent crosslinked or graft copolymers formed from an ampholytic ion pair copolymerized with other comonomers. The aforesaid crosslinked or graft superabsorbent copolymers are useful for absorbing aqueous electrolyte solutions.

Polymers for absorbing aqueous electrolyte solutions are used in numerous commercial and industrial applications. For example, polymers are used to improve the water absorbency of paper towels and disposable diapers.

Though known water absorbing polymers are highly absorbent to deionized water, they are dramatically less absorbent to aqueous electrolyte solutions such as salt water, brine, and urine. For example, hydrolyzed crosslinked polyacrylamide absorbs 1,024 grams of deionized water per gram of polymer, but only 25 grams of synthetic urine per gram of polymer. Crosslinked polyacrylate absorbs 423 grams of deionized water per gram of polymer, but only 10 grams of synthetic urine per gram of polymer. Hydrolyzed crosslinked polyacrylonitrile absorbs 352 grams of deionized water per gram of polymer, but only 25 grams of synthetic urine per gram of polymer. Analogous starch grafted copolymers generally have very poor absorbency to synthetic urine.

EP 0068189 discloses crosslinked copolymerizates of a) 2-acrylamido-2-methylpropanesulphonic acid or its alkali- and/or ammonium salts, b) acrylic acid and/or methacrylic acid or their alkali and/or ammonium salts and/or acrylamid and/or vinylpyrrolidone and c) at least one bifunctional crosslinking agent. Furthermore, the use of the copolymerizates for absorbing urine and other aqueous electrolyte solutions is shown.

GB-A-1524899 discloses random interpolymers derived from the polymerization of a comonomer mixture comprising an ester of an a, β- olefinically unsaturated carboxylic acid and a monohydric or polyhydric alcohol having a terminal quaternary ammonium group in the presence of a difunctional monomeric crosslinking agent.

FR-A-2582663 discloses a mixture of electrically charged particles which are agglomerated with a composition of a super-absorbent, hydrocolloidal polyelectrolyte.

It would be a valuable contribution to the art to develop polymers with high absorbency to aqueous electrolyte solutions such as tap water, salt water, brine, and urine. It also would be a valuable contribution to the art to develop inexpensive polymers with high absorbency to aqueous electrolyte solutions. The market for these types of polymers is large and the uses are numerous. Therefore, seemingly small improvements in the absorbency translate into large savings in the quantity of polymer required to absorb these aqueous electrolyte solutions and large savings to the consumer. Preferably such polymers should be biodegradable.

The polymers of the present invention comprise a polymer suitable for use as an absorbent of an aqueous electrolyte solution, said polymer being formed by copolymerization of the following components:
(a) an ampholytic ion pair monomer comprising
   (i) the ammonium cation 3-methacrylamidopropyldimethylammonium, 2-methacryloyloxyethyldimethylammonium or 2-methacryloyloxyethyldiethylammonium and
   (ii) a sulfonate anion which is 2-acrylamido-2-methylpropane sulfonate, 2-methacryloyloxyethane sulfonate, vinyl sulfonate, styrene sulfonate or a combination of two or more thereof; and
(b) at least one comonomer which is acrylamide, methacrylamide, acrylonitrile, acrylic acid, methacrylic acid, an alkali salt of acrylic acid, an alkali salt of methacrylic acid, 2-acrylamido-2-methylpropane sulfonic acid, an alkali salt of 2-acrylamido-2-methylpropane sulfonic acid, 2-methacryloyloxyethane sulfonic acid, an alkali salt of 2-methacryloyloxyethane sulfonic acid, N-vinyl-2-pyrrolidone, or an amine corresponding to the ammonium cation (a) (i), namely 3-methacrylamidopropyldimethylamine, 2-methacryloyloxyethyldimethylamine, or 2-methacryloyloxyethyldiethylamine, respectively, or a combination of two or more of said comonomers;
and wherein there is further included
(c) a crosslinking agent which has at least two polymerizable olefinic functionalities wherein each of the olefinic functionalities is suitable for crosslinking with polymer chains formed from components (a) and (b) or
(c') a polymer which is a polysaccharide, polypropylene, or polyethylene onto which the comonomer (b) is graft polymerized and with which there is further graft copolymerized the ampholytic ion pair monomer (a); wherein the comonomer component (b) and the ion pair monomer component (a) are provided in amounts which are effective to produce a highly absorbent copolymer.

A further aspect of the invention relates to a method of absorbing an aqueous electrolyte solution comprising the step of contacting the polymers of the present invention with an aqueous electrolyte solution.

The present invention provides polymers that are highly absorbent to aqueous electrolyte solutions. Typical aqueous electrolyte solutions include but are not limited to the group consisting of tap water, salt water, brine, and urine. The polymers of the present invention comprise a polymer suitable for use as an absorbent of an aqueous electrolyte solution, said polymer being formed by copolymerization of the following components:
(a) an ampholytic ion pair monomer comprising
   (i) the ammonium cation 3-methacrylamidopropyldimethylammonium, 2-methacryloyloxyethyldimethylammonium or 2-methacryloyloxyethyldiethylammonium and
   (ii) a sulfonate anion which is 2-acrylamido-2-methylpropane sulfonate, 2-methacryloyloxyethane sulfonate, vinyl sulfonate, styrene sulfonate or a combination of two or more thereof; and
(b) at least one comonomer which is acrylamide, methacrylamide, acrylonitrile, acrylic acid, methacrylic acid, an alkali salt of acrylic acid, an alkali salt of methacrylic acid, 2-acrylamido-2-methylpropane sulfonic acid, an alkali salt of 2-acrylamido-2-methylpropane sulfonic acid, 2-methacryloyloxyethane sulfonic acid, an alkali salt of 2-methacryloyloxyethane sulfonic acid, N-vinyl-2-pyrrolidone, or an amine corresponding to the ammonium cation (a) (i), namely 3-methacrylamidopropyldimethylamine, 2-methacryloyloxyethyldimethylamine, or 2-methacryloyloxyethyldiethylamine, respectively, or a combination of two or more of said comonomers;
and wherein there is further included
(c) a crosslinking agent which has at least two polymerizable olefinic functionalities wherein each of the olefinic functionalities is suitable for crosslinking with polymer chains formed from components (a) and (b) or
(c') a polymer which is a polysaccharide, polypropylene, or polyethylene onto which the comonomer (b) is graft polymerized and with which there is further graft copolymerized the ampholytic ion pair monomer (a); wherein the comonomer component (b) and the ion pair monomer component (a) are provided in amounts which are effective to produce a highly absorbent copolymer.

As used in this application, the term "alkali salts" is used generically, unless otherwise indicated, to mean alkali salts including but not limited to salts containing lithium, sodium, potassium, and ammonium cations.

As used in this application, the term "monomer" is used generically, unless otherwise indicated, to mean monomers, comonomers, termonomers, tetramonomers, etc. The term "comonomer" is used generically, unless otherwise indicated, to mean monomers, comonomers, termonomers, tetramonomers, etc. for polymers wherein there are at least two different monomers.

As used in this application, the term "polymer" is used generically, unless otherwise indicated, to mean homopolymers, copolymers, terpolymers, tetrapolymers, etc., and thus includes polymers prepared using any number of monomers. The term "copolymer" is used generically to mean polymers prepared using two or more different monomers.

Graft copolymers as used herein are polymers of one or more species of monomers connected to a main chain as a side chain, exclusive of branch point on the main chain. Side chains of a graft copolymer are distinguished from the main polymer chain by the monomer constitution of the side chain i.e., the side chains comprise units derived from at least one species of monomer different from those that supply the units of the main polymer chain. The main polymer chain as utilized in the present invention are homopolymeric and copolymeric polymer such as polysaccharide, polypropylene, polyethylene and other polyolefins. The side chains are formed of olefinic comonomers and ampholytic ion pairs.

The term "graft copolymerization" is used herein, unless otherwise indicated, to mean a copolymer which results from the formation of an active site or sites at one or more points on the main chain of a polymer molecule other than its end and exposure to at least one other monomer.

Polymer or copolymer which may be used as main chains in the practice of the present invention include polysaccharides, polypropylene, polyethylene and other polyolefins. Polysaccharides suitable for the practice of the present invention include starches, celluloses and glycogens. Common sources of cellulose include but are not limited to cotton, linen, rayon, wood pulp and cellulose xanthane. Currently, cotton gauze is preferred. Suitable starches included swollen amylose and amylopectin starches. For the practice of the present invention, these starches should be swollen by heating the starch in water to substantially dissolve the starch granules. Preferably starches used in the present invention will have less than 30 weight percent amylose based on the weight of the dry starch before graft. The preferred starch for use in grafting is soluble starch flour within the range of from about 0 to about 20 weight percent amylose content. Polypropylene polymer suitable for use as main polymer chain include polypropylene homopolymers, polypropylene copolymers and polypropylene block-copolymers. Polyethylene polymers suitable for use as a main polymer chain include polyethylene homopolymer, polyethylene copolymers and polyethylene block-copolymers. Preferably the synthetic polymers listed above will be utilized in the form of filaments or thin sheets so that a high surface area to mass will be provided for grafting the comonomers and ampholytic ion pair onto. Filaments utilized for grafting will preferably have a denier ranging from 0.11 to 2.2 tex (1 to 20 denier) and most preferably from in the range of 0.11 to 0.88 tex (1 to 8 denier).

As used in this application, the term "hydrolysis" is used generically, unless otherwise indicated, to include hydrolysis of nitrile functionalities and hydrolysis of amide functionalities. These hydrolysis reactions are loosely referred to in the art as "saponification." Hydrolysis of these functionalities may occur under acidic or basic conditions. Under basic hydrolysis conditions, the term may also include, unless otherwise indicated, neutralization of carboxylic acid and sulfonic acid functionalities.

The ampholytic ion pair monomer used in the present invention may be prepared by titrating an aqueous solution of a sulfonic acid monomer to pH7 with the amine corresponding to the ammonium cation at a temperature of about 0-15 C. The resulting aqueous solution containing the ampholytic ion pair may be purified by contacting the aqueous solution one or more times with small quantities of activated charcoal. The concentration of the ampholytic ion pair in the aqueous solution may be determined by evaporatively drying a known amount of the aqueous solution and weighing the residue.

Alternatively the ampholytic ion pair monomer for use in the preparation of the present invention may be prepared by methods which are well known to those skilled in the art. For example, the ampholytic ion pair monomers can be prepared by reacting the amine corresponding to the ammonium cation, e.g., 3-methacrylamidopropyldimethylamine, with the sulfonic acid corresponding to the sulfonate anion, e.g., commercially available 2-acrylamido-2-methylpropane sulfonic acid or 2-methacryloyloxyethane sulfonic acid, in anhydrous tetrahydrofuran. See J.C. Salamone, C.C. Tsai, A.P. Olson, and A.C. Watterson, Adv. Chemical Series, Volume 187, pages 337-346.

The olefinic comonomers can include but are not limited to the group consisting of acrylamide, methacrylamide, acrylonitrile, acrylic acid, methacrylic acid, alkali salts of acrylic acid, alkali salts of methacrylic acid, 3-methacrylamidopropyldimethylamine, 2-acrylamido-2-methylpropane sulfonic acid, alkali salts of 2-acrylamido-2-methylpropane sulfonic acid, 2-methacryloyloxyethane sulfonic acid, alkali salts of 2-methacryloyloxyethane sulfonic acid, N-vinyl-2-pyrrolidone and combinations of two or more thereof. All these suitable olefinic comonomers are believed to be commercially available.

Suitable crosslinking agents can include but are not limited to the group consisting of N,N-diallylmethacrylamide, diallylamine, N,N-bisacrylamidoacetic acid, N,N'-bisacrylamidoacetic acid methylester, N,N'-methylenebisacrylamide (methylene-bis-acrylamide), N,N-benzylidenebisacrylamide, allylacrylate, diisopropenylbenzene, diallyl succinate, ethylene glycol diacrylate, diallylacrylamide, divinylbenzene, and combinations of two or more thereof. All these suitable crosslinking agents are believed to be commercially available.

The polymers of the present invention formed from components (a),(b) and (c) were generally prepared by mixing the various monomers in the desired stoichiometric ratios in aqueous solution and then initiating the free-radical copolymerization.

The polymers of the present invention formed from components (a), (b), and (c') were generally prepared in a two step process, though a single graft copolymerizing step or more than two grafting and polymerizing steps may be advantageously employed. The purpose of the two step process is to provide a first grafted polymer wherein the grafted comonomer side chains are more reactive to the polymerization of the ampholytic ion pair monomer. Some systems may be reactive enough so that a two step process is not necessary to provide grafted copolymers which are highly absorbent to aqueous electrolyte solutions. Alternatively, the multiple step process may be advantageously employed to control the proportions of monomers and relative lengths of the block copolymer chains by graft copolymerizing the various monomers in the desired stoichiometric ratios at the appropriate step of the process.

In the preparation of polysaccharide graft ammonium/sulfonate copolymers it is preferred that as a first step, at least one of the comonomers is graft copolymerized onto a polysaccharide, to produce a first polysaccharide graft copolymer. Then in a second step, the ampholytic ion pair is graft copolymerized onto the polysaccharide or the ampholytic ion pair is polymerized onto the grated comonomer side chains. At the second or any subsequent graft copolymerizing step, the ampholytic ion pair monomer may be copolymerized with at least one other comonomer. At the second step or any subsequent graft copolymerizing step, the ampholytic ion pair monomer may be copolymerized with at least one comonomer which has a polymerizable olefinic functionality selected from the group consisting of acrylamide (also referred to as AM), methacrylamide, acrylonitrile (also referred to as AN), acrylic acid (also referred to as AA), methacrylic acid, alkali salts of acrylic acid (also referred to as X-AA), alkali salts of methacrylic acid, 3-methacrylamidopropyldimethylamine, 2-acrylamido-2-methylpropane sulfonic acid, alkali salts of 2-acrylamido-2-methylpropane sulfonic acid, 2-methacryloyloxyethane sulfonic acid, alkali salts of 2-methacryloyloxyethane sulfonic acid, N-vinyl-2-pyrrolidone and any combination of two or more thereof.

The polymerization of the ampholytic ion pair may require a higher temperature than the polymerization of some of the other comonomers. Therefore, for the polymerization of the ampholytic ion pair it is desirable to perform the polymerization at temperatures in the range of from 0°C. to 90°C. and preferably in the range of from 40°C. to 70°C. Those skilled in the art will recognize that the temperatures at which the polymerization is carried out should be varied to allow the various monomers and comonomer to react completely within a reasonable period of time for the method of polymerization utilized.

Most graft copolymerization methods for olefinic monomers involve the creation of reactive sites (for example free-radicals) on the main polymer chain. These reactive sites then serve to initiate the copolymerization of the other monomers onto the main copolymer chain. Free-radicals reactive sites on the main chain generally are produced by high energy radiation or chemical initiation. A common chemical means for creating these free-radicals within polysaccharide polymers and polypropylene polymers is with a chemical oxidation-reduction system. Examples of such oxidation-reduction systems include but are not limited to oxidation-reduction systems selected from the group consisting of ceric ammonium nitrate/nitric acid, ceric ammonium sulfate/sulfuric acid, potassium permanganate/oxalic acid, hydrogen peroxide/ferrous alkali salts, hydrogen peroxide/ascorbic acid and amine/persulfate. Common irradiation means for producing free radicals on the main polymer chain is by utilizing a gamma radiation source (i.e. cobalt 60) or an electron beam.

The copolymerization of the ampholytic ion pair monomer with the olefinic comonomer and the (c) crosslinking agent or with the olefinic comonomer onto the (c') grafted comonomer side chains can be achieved by any of the well known free-radical polymerization techniques in solution, suspension, or emulsion environment. Well known azo compounds commonly employed to initiate free radical polymerization reactions include 2,2'-azobis(N,N'-dimethylisobutyramidine) dihydrochloride, azobisisobutyronitrile, 4,4'-azobis(4-cyanovaleric acid), 2,2'-azobis(2,4-dimethyl-4-methyoxyvaleronitrile), 2,2'-azobis(2,4-dimethylvaleronitrile), 2,2'-azobis(2-amidinopropane)dihydrochloride, 2-t-butylazo-2-cyano-4-methoxy-4-methylpentane, 2-t-butylazo-2-cyano-4 methylpentane, and 4-t-butylazo-4-cyanovaleric acid. Well known inorganic peroxide compounds commonly employed to initiate free radical polymerization reactions include hydrogen peroxide, alkali metal persulfates, alkali metal perborates, alkali metal perphosphates, and alkali metal percarbonates. Well known organic peroxide compounds commonly employed to initiate free radical polymerization reactions include lauryl peroxide, 2,5-dimethyl-2,5-bis(2-ethylhexanylperoxy)hexane, t-butylperoxypivalate, t-butylperoctoate, p-menthane hydroperoxide, and benzoylperoxide. The compound t-butylhyponitrite is a well known alkyl hyponitrite commonly employed to initiate free radical polymerization reactions. Furthermore, ultraviolet light is commonly employed to initiate free radical polymerization reactions with olefinic monomers. In addition, such other methods of copolymerization as would have occurred to one skilled in the art may be employed, and the present invention is not limited to the particular method of preparing the polymer set out herein. The appropriate conditions under which the polymerization reactions described above may be carried out are well known in the art.

Optionally the graft copolymers of the present invention can also be crosslinked with the crosslinking agent (c) described above. The crosslinking agent should be admixed with the monomer or comonomers when the side chains, are being formed from the main polymer chain. The amount of crosslinking agent admixed with the monomers or comonomers will be in the range of from 0.01 to 0.2 weight percent of total weight of monomers and comonomers in the graft copolymerization reaction.

The copolymers of the invention containing an olefinic comonomer with amide, nitrile, carboxylic acid, or sulfonic acid functionalities or crosslinking agent with amide, nitrile, carboxylic acid, or sulfonic acid functionalities can optionally be at least partially hydrolyzed and/or neutralized by heating with aqueous base such as aqueous sodium hydroxide or aqueous potassium hydroxide. The degree of hydrolysis and/or neutralization can be controlled by stoichiometrically limiting the amount of base relative to the amount of amide, nitrile, carboxylic acid, and sulfonic acid functionalities. If the hydrolysis is carried out under acidic conditions, the amide and nitrile functionalities can be converted to carboxylic acid functionalities without neutralizing the carboxylic acid or sulfonic acid functionalities of the polymer.

The broadest range for the compositions of the inventive crosslinked ammonium/sulfonate copolymers formed from components (a), (b) and (c) is an effective amount of each of the ampholytic ion pair monomer, olefinic comonomer, and crosslinking agent to produce a polymer highly absorbent to aqueous electrolyte solutions. The preferred ranges for the compositions of the inventive polymers given in Tables I-IX reflect the relative stoichiometric amount in mole percent based on the total number of moles of all the various monomers mixed together before the copolymerization. The ratio of the crosslinking agent to the other monomers is based on the total number of moles of the ampholytic ion pair and the comonomers. The actual composition of the polymers of the present invention produced by the copolymerization reaction may vary slightly from the stoichiometric mixture before the copolymerization depending on the reaction conditions.

The broad and preferred ranges for the compositions of the inventive crosslinked MPDMA/sulfonate, MEDMA/sulfonate and MEDEA/sulfonate copolymers are given in Tables I, IV and VII, respectively. These broadly preferred ranges for the compositions of the present invention are based on the experimental data provided in Example V, Tables XXII-XXVII, for those polymer compositions which produce an absorbency of at least 70 gram of synthetic urine per gram of inventive crosslinked MPDMA/sulfonate MEDMA/sulfonate or MEDEA/sulfonate copolymer.

**Table I**

| Broad Compositions For Inventive MPDMA/sulfonate Polymers | | | | | | |
|---|---|---|---|---|---|---|
| | MPDMA/sulfonate | AM | AN | AA | X-AA | LINK |
| | ---------------------MOLE PERCENT ------------------ | | | | | |
| broad | 10-30 | 70-90 | -- | -- | -- | 0.01-0.3 |
| preferred | 10-25 | 75-90 | -- | -- | -- | 0.01-0.2 |
| broad | 3-35 | -- | 65-97 | -- | -- | 0.01-0.3 |
| preferred | 5-25 | -- | 75-95 | -- | -- | 0.01-0.2 |
| broad | 1-30 | -- | -- | -- | 70-99 | 0.01-0.3 |
| preferred | 1-10 | -- | -- | -- | 90-99 | 0.01-0.2 |
| broad | 1-20 | 10-25 | -- | -- | 55-89 | 0.01-0.3 |
| preferred | 1.5-15 | 10-20 | -- | -- | 65-88.5 | 0.01-0.2 |
| MPDMA/sulfonate = 3-methacrylamidopropyldimethylammonium cation/a sulfonate anion selected from the group consisting of 2-acrylamido-2-methylpropane sulfonate, 2-methacryloyloxyethane sulfonate, and any combinations thereof AM = Acrylamide AN = Acrylonitrile AA = Acrylic Acid X-AA = Alkali Salt of Acrylic Acid (Acrylate) Link = Crosslinking Agent | | | | | | |

The more preferred and most preferred ranges for the compositions of the inventive crosslinked MPDMA/AMPS copolymers are given in Table II. These more preferred and most preferred ranges for the compositions of the present invention are based on the experimental data provided in Example V, Table XXII, for those polymer compositions which produce an absorbency of at least 70 gram of synthetic urine per gram of inventive MPDMA/AMPS polymer.

**Table II**

| Preferred Compositions For Inventive MPDMA/AMPS Polymers | | | | | | |
|---|---|---|---|---|---|---|
| | MPDMA/AMPS | AM | AN | AA | X-AA | LINK |
| | -------------------MOLE PERCENT -------------- | | | | | |
| more preferred | 10-30 | 70-90 | -- | -- | -- | 0.01-0.2 |
| most preferred | 10-25 | 75-90 | -- | -- | -- | 0.01-0.1 |
| more preferred | 3-35 | -- | 65-97 | -- | -- | 0.01-0.2 |
| most preferred | 5-25 | -- | 75-95 | -- | -- | 0.01-0.1 |
| more preferred | 1-15 | -- | -- | -- | 85-99 | 0.01-0.2 |
| most preferred | 1-10 | -- | -- | -- | 90-99 | 0.01-0.1 |
| more preferred | 1.5-15 | 10-21 | -- | -- | 64-88.5 | 0.01-0.2 |
| most preferred | 6-10 | 10-15 | -- | -- | 75-84 | 0.01-0.1 |
| MPDMA/AMPS = 3-methacrylamidopropyldimethylammonium cation/2-acrylamido-2-methylpropane sulfonate AM = Acrylamide AN = Acrylonitrile AA = Acrylic Acid X-AA = Alkali Salt of Acrylic Acid (Acrylate) LINK = Crosslinking Agent | | | | | | |

The more preferred and most preferred ranges for the compositions of the inventive crosslinked MPDMA/MES copolymers are given in Table III. These more preferred and most preferred ranges for the compositions of the present invention are based on the experimental data provided in Example V, Table XXIII, for those polymer compositions which produce an absorbency of at least 70 gram of synthetic urine per gram of inventive MPDMA/MES polymer.

**Table III**

| Preferred Compositions For Inventive MPDMA/MES Polymers | | | | | | |
|---|---|---|---|---|---|---|
| | MPDMA/MES | AM | AN | AA | X-AA | LINK |
| | -------------------MOLE PERCENT -------------- | | | | | |
| more preferred | 10-30 | 70-00 | -- | -- | -- | 0.01-0.2 |
| most preferred | 10-25 | 75-90 | -- | -- | -- | 0.01-0.1 |
| more preferred | 3-35 | -- | 65-97 | -- | -- | 0.01-0.2 |
| most preferred | 5-25 | -- | 75-95 | -- | -- | 0.01-0.1 |
| more preferred | 1-25 | -- | -- | -- | 75-99 | 0.01-0.2 |
| most preferred | 1-6 | -- | -- | -- | 94-99 | 0.01-0.1 |
| more preferred | 1.5-15 | 10-21 | -- | -- | 64-88.5 | 0.01-0.2 |
| most preferred | 6-10 | 10-15 | -- | -- | 75-84 | 0.01-0.1 |
| MPDMA/MES = 3 methacrylamidopropyldimethylammonium cation/2-methacryloyloxyethane sulfonate AM = Acrylamide AN = Acrylonitrile AA = Acrylic Acid X-AA = Alkali Salt of Acrylic Acid (Acrylate) LINK = Crosslinking Agent | | | | | | |

**Table IV**

| Broad Compositions For Inventive MEDMA/sulfonate Polymers | | | | | | |
|---|---|---|---|---|---|---|
| | MEDMA/sulfonate | AM | AN | X-AMPS | X-AA | LINK mole ratio* |
| | ---------------MOLE PERCENT -------------- | | | | | |
| broad | 3-50 | 50-97 | -- | -- | -- | 0.01-0.3 |
| preferred | 5-50 | 50-95 | -- | -- | -- | 0.01-0.2 |
| broad | 6-25 | -- | 75-95 | -- | -- | 0.01-0.3 |
| preferred | 10-20 | -- | 80-90 | -- | -- | 0.01-0.2 |
| broad | 10-25 | -- | -- | 75-90 | -- | 0.01-0.3 |
| preferred | 15-20 | -- | -- | 80-85 | -- | 0.01-0.2 |
| broad | 3-6 | -- | -- | -- | 94-97 | 0.01-0.3 |
| preferred | 3-6 | -- | -- | -- | 94-97 | 0.01-0.2 |
| broad | 1-10 | 13-25 | -- | 65-86 | -- | 0.01-0.3 |
| preferred | 3-6 | 13-20 | -- | 74-84 | -- | 0.01-0.2 |
| broad | 1-30 | 10-50 | -- | -- | 20-89 | 0.01-0.3 |
| preferred | 1-25 | 13-50 | -- | -- | 25-86 | 0.01-0.2 |
| MEDMA/sulfonate = 2-methacryloyloxyethyldimethylammonium cation/a sulfonate anion selected from the group consisting of 2-acrylamido-2-methylpropane sulfonate, 2-methacryloyloxyethane sulfonate, vinyl sulfonate, styrene sulfonate and combinations of two or more thereof AM = Acrylamide AN = Acrylonitrile X-AMPS = Alkali salt of 2-acrylamido-2-methylpropane sulfonate (AMPS is a trademark of Lubrizol Corporation) X-AA = Alkali Salt of Acrylic Acid (Acrylate) LINK = Crosslinking Agent | | | | | | |

| | | | | | | |
|---|---|---|---|---|---|---|
| *mole ratio = mole crosslinking agent per 100 mole of the ampholytic ion pair monomer and the comonomers. | | | | | | |

The more preferred and most preferred ranges for the compositions of the inventive crosslinked MEDMA/AMPS copolymers are given in Table V. These more preferred and most preferred ranges for the compositions of the present invention are based on the experimental data provided in Example V, Table XXIV, for those polymer compositions which produce an absorbency of at least 70 gram of synthetic urine per gram of inventive MEDMA/AMPS polymer.

**Table V**

| Preferred Compositions For Inventive MEDMA/AMPS Polymers | | | | | | |
|---|---|---|---|---|---|---|
| | MEDMA/AMPS | AM | AN | X-AMPS | X-AA | LINK mole ratio* |
| | ----------MOLE PERCENT ------------ | | | | | |
| more preferred | 3-20 | 80-97 | -- | -- | -- | 0.01-0.2 |
| most preferred | 5-10 | 90-95 | -- | -- | -- | 0.01-0.1 |
| more preferred | 10-20 | -- | 80-90 | -- | -- | 0.01-0.2 |
| most preferred | 10-20 | -- | 80-90 | -- | -- | 0.01-0.1 |
| more preferred | 10-25 | -- | -- | 75-90 | -- | 0.01-0.2 |
| most preferred | 15-20 | -- | -- | 80-85 | -- | 0.01-0.1 |
| more preferred | 3-6 | -- | -- | -- | 94-97 | 0.01-0.2 |
| most preferred | 3 | -- | -- | -- | 97 | 0.01-0.1 |
| more preferred | 1-10 | 13-25 | -- | 65-86 | -- | 0.01-0.2 |
| most preferred | 3-6 | 13-20 | -- | 74-84 | -- | 0.01-0.1 |
| more preferred | 1-6 | 13-26 | -- | -- | 68-86 | 0.01-0.2 |
| most preferred | 1-3 | 13-26 | -- | -- | 71-86 | 0.01-0.1 |
| MEDMA/AMPS = 2-methacryloyloxyethyldimethylammonium cation/2-acrylamido-2-methylpropane sulfonate AM = Acrylamide AN = Acrylonitrile X-AMPS = Alkali salt of 2-acrylamido-2-methylpropane sulfonate (AMPS is a trademark of Lubrizol Corporation) X-AA = Alkali Salt of Acrylic Acid (Acrylate) LINK = Crosslinking Agent | | | | | | |

| | | | | | | |
|---|---|---|---|---|---|---|
| *mole ratio = mole crosslinking agent per 100 mole of the ampholytic ion pair monomer and the comonomers. | | | | | | |

The more preferred and most preferred ranges for the compositions of the inventive crosslinked MEDMA/MES copolymers are given in Table VI. These more preferred and most preferred ranges for the compositions of the present invention are based on the experimental data provided in Example V, Table XXV, for those polymer compositions which produce an absorbency of at least 70 gram of synthetic urine per gram of inventive crosslinked MEDMA/MES copolymer.

**Table VI**

| Preferred Compositions For Inventive MEDMA/MES Polymers | | | | | | |
|---|---|---|---|---|---|---|
| | MEDMA/MES | AM | AN | X-AMPS | X-AA | LINK mole ratio* |
| | ----------MOLE PERCENT------------ | | | | | |
| more preferred | 10-50 | 50-90 | -- | -- | -- | 0.01-0.2 |
| most preferred | 10-15 | 85-95 | -- | -- | -- | 0.01-0.1 |
| more preferred | 15-20 | -- | 80-85 | -- | -- | 0.01-0.2 |
| most preferred | 15-20 | -- | 80-85 | -- | -- | 0.01-0.1 |
| more preferred | 10-25 | -- | -- | 75-90 | -- | 0.01-0.2 |
| most preferred | 15-20 | -- | -- | 80-85 | -- | 0.01-0.1 |
| more preferred | 3 | -- | -- | -- | 97 | 0.01-0.2 |
| most preferred | 3 | -- | -- | -- | 97 | 0.01-0.1 |
| more preferred | 1-10 | 13-25 | -- | 65-86 | -- | 0.01-0.2 |
| most preferred | 3-6 | 13-20 | -- | 74-84 | -- | 0.01-0.1 |
| more preferred | 3-25 | 13-47 | -- | -- | 28-84 | 0.01-0.2 |
| most preferred | 3-15 | 13-35 | -- | -- | 50-84 | 0.01-0.1 |
| MEDMA/MES = 2-methacryloyloxyethyldimethylammonium cation/ 2-methacryloyloxyethane sulfonate AM = Acrylamide AN = Acrylonitrile X-AMPS = Alkali salt of 2-acrylamido-2-methylpropane sulfonate (AMPS is a trademark of Lubrizol Corporation) X-AA = Alkali Salt of Acrylic Acid (Acrylate) LINK = Crosslinking Agent | | | | | | |

| | | | | | | |
|---|---|---|---|---|---|---|
| * mole ratio = mole crosslinking agent per 100 mole of the ampholytic ion pair monomer and the comonomers. | | | | | | |

**Table VII**

| Broad And Preferred Ranges Of Compositions For Inventive MEDEA/sulfonate Polymers | | | | | | |
|---|---|---|---|---|---|---|
| | MEDEA/sulfonate | AM | AN | AA | X-AA | LINK mole ratio* |
| | ---------------MOLE PERCENT ----------- | | | | | |
| broad | 3-50 | 50-97 | -- | -- | -- | 0.01-0.3 |
| preferred | 3-30 | 70-97 | -- | -- | -- | 0.03-0.2 |
| broad | 3-30 | -- | 70-97 | -- | -- | 0.01-0.3 |
| preferred | 5-25 | -- | 75-95 | -- | -- | 0.03-0.2 |
| broad | 3-6 | -- | -- | -- | 94-97 | 0.01-0.3 |
| preferred | 3 | -- | -- | -- | 97 | 0.03-0.2 |
| broad | 1-55 | 10-55 | -- | -- | 32-89 | 0.01-0.3 |
| preferred | 3-50 | 13-50 | -- | -- | 37-84 | 0.03-0.2 |
| MEDEA/sulfonate = 2-methacryloyloxyethyldiethylammonium cation/ a sulfonate anion selected from the group consisting of 2-acrylamido-2-methylpropane sulfonate, 2-methacryloyloxyethane sulfonate, and any combinations thereof AM = Acrylamide AN = Acrylonitrile AA = Acrylic Acid X-AA = Alkali Salt of Acrylic Acid (Acrylate) LINK = Cross-Linking Agent | | | | | | |

| | | | | | | |
|---|---|---|---|---|---|---|
| * mole ratio = mole crosslinking agent per 100 mole of the ampholytic ion pair monomer and the comonomers. | | | | | | |

The more preferred and most preferred ranges for the compositions of the inventive crosslinked MEDEA/AMPS copolymers are given in Table VIII. These more preferred and most preferred ranges for the compositions of the present invention are based on the experimental data provided in Example V, Table XXVI, for those polymer compositions which produce an absorbancy of at least 70 gram of synthetic urine per gram of inventive MEDEA/AMPS polymer.

**Table VIII**

| Preferred Compositions For Inventive MEDEA/AMPS Polymers | | | | | | |
|---|---|---|---|---|---|---|
| | MEDEA/AMPS | AM | AN | AA | X-AA | LINK mole ratio* |
| | -----------MOLE PERCENT------------ | | | | | |
| more preferred | 3-30 | 70-97 | -- | -- | -- | 0.01-0.2 |
| most preferred | 5-20 | 80-95 | -- | -- | -- | 0.03-0.2 |
| more preferred | 3-25 | -- | 75-97 | -- | -- | 0.01-0.2 |
| most preferred | 5-20 | -- | 80-95 | -- | -- | 0.03-0.2 |
| more preferred | 3-6 | -- | -- | -- | 94-97 | 0.01-0.2 |
| most preferred | 3 | -- | -- | -- | 97 | 0.03-0.2 |
| more preferred | 3-15 | 15-30 | -- | -- | 55-82 | 0.01-0.2 |
| most preferred | 3-10 | 20-25 | -- | -- | 70-77 | 0.03-0.2 |
| MEDEA/AMPS = 2-methacryloyloxyethyldiethylammonium cation/ 2-acrylamido-2-methylpropane sulfonate AM = Acrylamide AN = Acrylonitrile AA = Acrylic Acid X-AA = Alkali Salt of Acrylic Acid (Acrylate) LINK = Cross-Linking Agent | | | | | | |

| | | | | | | |
|---|---|---|---|---|---|---|
| * mole ratio = mole crosslinking agent per 100 mole of the ampholytic ion pair monomer and the comonomers. | | | | | | |

The more preferred and most preferred ranges for the compositions of the inventive crosslinked MEDEA/MES copolymers are given in Table IX. These more preferred and most preferred ranges for the compositions of the present invention are based on the experimental data provided in Example V, Table XXVII, for those polymer compositions which produce an absorbency of at least 70 gram of synthetic urine per gram of inventive crosslinked MEDEA/MES copolymer.

**Table IX**

| Preferred Compositions For Inventive MEDEA/MES Polymers | | | | | | |
|---|---|---|---|---|---|---|
| | MEDEA/MES | AM | AN | AA | X-AA | LINK mole ratio* |
| | -----------MOLE PERCENT ------------ | | | | | |
| more preferred | 3-50 | 50-97 | -- | -- | -- | 0.01-0.2 |
| most preferred | 3-30 | 70-97 | -- | -- | -- | 0.03-0.2 |
| more preferred | 3-30 | -- | 70-97 | -- | -- | 0.01-0.2 |
| most preferred | 6-25 | -- | 75-94 | -- | -- | 0.03-0.2 |
| more preferred | 3-6 | -- | -- | -- | 94-97 | 0.01-0.2 |
| most preferred | 3 | -- | -- | -- | 97 | 0.03-0.2 |
| more preferred | 1-55 | 10-55 | -- | -- | 32-89 | 0.01-0.2 |
| most preferred | 3-50 | 13-50 | -- | -- | 37-84 | 0.03-0.2 |
| MEDEA/MES = 2-methacryloyloxyethyldiethylammonium cation/ 2-methacryloyloxyethane sulfonate AM = Acrylamide AN = Acrylonitrile AA = Acrylic Acid X-AA = Alkali Salt of Acrylic Acid (Acrylate) LINK = Cross-Linking Agent | | | | | | |

| | | | | | | |
|---|---|---|---|---|---|---|
| * mole ratio = mole crosslinking agent per 100 mole of the ampholytic ion pair monomer and the comonomers. | | | | | | |

In preparing the copolymers from components (a), (b) and (c'), the relative amount of the main polymer chain to the total weight of the comonomer and ampholytic ion pair can be chosen to provide a graft copolymer of variable absorbency. However, it is preferred that the main polymer chain constitutes in the range of from about 1 to about 50 weight percent of the total weight of comonomers, ampholytic ion pair and main polymer chain present and most preferably it is preferred that the amount of main polymer chain be in the range from about 5 to about 30 weight percent. The mole percent of comonomer and ampholytic ion pair which may be graft copolymerized onto the main polymer chain is given in Tables X-XV (these mole percents are based on the total moles of the comonomers and ampholytic ion pair totaling a 100 percent).

**Table X**

| Broad Comonomer Compositions for the MPDMA/sulfonate Graft Copolymers | | | | |
|---|---|---|---|---|
| | MPDMA/sulfonate | AM | AN | X-AA |
| | ----------------------MOLE PERCENT------------------- | | | |
| broad | 2-25 | ---- | 98-75 | ---- |
| broad | 2-20 | 98-80 | ---- | ---- |
| broad | 2-20 | ---- | ---- | 98-80 |
| MPDMA/sulfonate = 3-methacrylamidopropyldimethylammonium cation/ a sulfonate anion selected from the group consisting of 2-acrylamido-2-methylpropane sulfonate, 2-methacryloyloxyethane sulfonate, and any combinations thereof AM = Acrylamide AN = Acrylonitrile X-AA = Alkali Salt of Acrylic Acid | | | | |

**Table XI**

| Preferred Comonomer Compositions for the MPDMA/AMPS® Graft Copolymers | | | | | |
|---|---|---|---|---|---|
| | MPDMA/AMPS® | MPDMA/MES | AM | AN | X-AA |
| | ------------------------MOLE PERCENT----------- | | | | |
| preferred | 5-25 | ----- | ----- | 75-95 | ----- |
| preferred | ----- | 5-20 | ----- | 80-95 | ----- |
| preferred | 5-20 | ----- | 95-80 | ----- | ----- |
| preferred | ----- | 10-20 | 90-80 | ----- | ----- |
| preferred | 3-15 | ----- | ----- | ----- | 97-85 |
| preferred | ----- | 3-10 | ----- | ----- | 97-90 |
| MPDMA/MES = 3-methacrylamidopropyldimethylammonium cation/ 2-methacryloyloxyethane sulfonate MPDMA/AMPS® = 3-methacrylamidopropyldimethylammonium cation/ 2-acrylamido-2-methylpropane sulfonate AM = Acrylamide AN = Acrylonitrile X-AA = Alkali Salt of Acrylic Acid (Acrylate) | | | | | |

**Table XII**

| Broad Comonomer Compositions for the MEDMA/sulfonate Graft Copolymers | | | | |
|---|---|---|---|---|
| | MEDMA/sulfonate | AM | AN | X-AA |
| | ----------------------MOLE PERCENT-------------------- | | | |
| broad | 2-25 | ---- | 98-75 | ---- |
| broad | 2-20 | 98-80 | ---- | ---- |
| broad | 2-20 | ---- | ---- | 98-80 |
| MEDMA/sulfonate = 2-methacryloyloxyethyldimethylammonium cation/ a sulfonate anion selected from the group consisting of 2-acrylamido-2-methylpropane sulfonate, 2-methacryloyloxyethane sulfonate, and any combinations thereof AM = Acrylamide AN = Acrylonitrile X-AA = Alkali Salt of Acrylic Acid | | | | |

**Table XIII**

| Preferred Comonomer Compositions for the MEDMA/AMPS® Graft Copolymers | | | | | |
|---|---|---|---|---|---|
| | MEDMA/AMPS® | MEDMA/MES | AM | AN | X-AA |
| | ------------------------MOLE PERCENT--------------- | | | | |
| preferred | 7-20 | ----- | ----- | 93-80 | ----- |
| preferred | ----- | 10-25 | ----- | 75-90 | ----- |
| preferred | 5-15 | ----- | 95-85 | ----- | ----- |
| preferred | ----- | 10-15 | 90-85 | ----- | ----- |
| preferred | 3-15 | ----- | ----- | ----- | 97-85 |
| preferred | ----- | 3-10 | ----- | ----- | 97-90 |
| MEDMA/MES = 2-methacryloyloxyethyldimethylammonium cation/ 2-methacryloyloxyethane sulfonate MEDMA/AMPS® = 2-methacryloyloxyethyltrimethylammonium cation/ 2-acrylamido-2-methylpropane sulfonate AM = Acrylamide AN = Acrylonitrile X-AA = Alkali Salt of Acrylic Acid (Acrylate) | | | | | |

**Table XIV**

| Broad Comonomer Compositions for the MEDEA/sulfonate Graft Copolymers | | | | |
|---|---|---|---|---|
| | MEDEA/sulfonate | AM | AN | X-AA |
| | ----------------------MOLE PERCENT------------------- | | | |
| broad | 2-25 | ---- | 98-75 | ---- |
| broad | 2-20 | 98-80 | ---- | ---- |
| broad | 2-20 | ---- | ---- | 98-80 |
| MEDEA/sulfonate = 2-methacryloyloxyethyldiethylammonium cation/ a sulfonate anion selected from the group consisting of 2-acrylamido-2-methylpropane sulfonate, 2-methacryloyloxyethane sulfonate and any combinations thereof AM = Acrylamide AN = Acrylonitrile X-AA = Alkali Salt of Acrylic Acid | | | | |

**Table XV**

| Preferred Comonomer Compositions for the MEDEA/AMPS ® Graft Copolymers | | | | | |
|---|---|---|---|---|---|
| | MEDEA/AMPS® | MEDEA/MES | AM | AN | X-AA |
| | ------------------------MOLE PERCENT-------------- | | | | |
| preferred | 5-20 | ----- | ----- | 95-80 | ----- |
| preferred | ----- | 5-20 | ----- | 95-80 | ----- |
| preferred | 5-15 | ----- | 95-85 | ----- | ----- |
| preferred | ----- | 10-15 | 90-85 | ----- | ----- |
| preferred | 3-15 | ----- | ----- | ----- | 97-85 |
| preferred | ----- | 3-10 | ----- | ----- | 97-90 |
| MEDEA/MES = 2-methacryloyloxyethyldiethylammonium cation/ 2-methacryloyloxyethane sulfonate MEDEA/AMPS® = 2-methacryloyloxyethyldiethylammonium cation/ 2-acrylamido-2-methylpropane sulfonate AM = Acrylamide AN = Acrylonitrile X-AA = Alkali Salt of Acrylic Acid (Acrylate) | | | | | |

The polysaccharide grafted polymers of the present invention should be highly biodegradable because the main chain of the graft copolymer is highly biodegradable.

A further aspect of the invention relates to a method of absorbing aqueous electrolyte solutions comprising the step of contacting the polymers of the present invention with the aqueous solution. Typical aqueous electrolyte solutions include but are not limited to electrolyte solutions selected from the group consisting of tap water, salt water, brine, and urine. For the purpose of this invention, tap water is defined to have an electrolyte concentration of less than 500 ppm of dissolved electrolytes, urine is defined to have an electrolyte concentration of from greater than 500 ppm to at most 10,000 ppm of dissolved electrolytes, salt water is defined to have an electrolyte concentration from greater than 10,000 ppm to at most 34,000 ppm and brine is defined to have an electrolyte concentration of from greater than 34,000 ppm to the saturation point of the solution.

The following examples are intended to illustrate the advantages of this invention but are not intended to unduly limit this invention.

### Example I

The control data in Table XVI demonstrates that although known crosslinked polymers are highly absorbent to deionized water, they are dramatically less absorbent to aqueous electrolyte solutions such as salt water and urine. Polysaccharide grafted polymers, however, according to their inherent nature, are normally much less absorbent to aqueous liquids. The polysaccharide substrate, which comprises a large portion of the material, is very poorly absorbent to aqueous liquids of all kinds. This control data can be used to show that the polysaccharide grafted ammonium/sulfonate copolymers of the present invention can effectively compete with these known crosslinked polymers and exceed the absorbency of these known crosslinked polymers. Furthermore, these known crosslinked polymers have questionable biodegradability. Known polymer composition include crosslinked polyacrylamide, partially saponified crosslinked polyacrylamide, crosslinked polyacrylonitrile, partially saponified crosslinked acrylonitrile, crosslinked polyacrylic acid, neutralized crosslinked polyacrylic acid, crosslinked polyacrylate, and polymers thereof with sodium 2-acrylamido-2-methylpropane sulfonate. The best of these known polymers absorbs up to about 60 grams of urine per gram of polymer, and most of the known polymers absorb much less than 50 grams of urine per gram of polymer.

The polymers of the control data were prepared by mixing the monomers in the proportions given in Table XVI in an aqueous solution of deionized water. The monomers were present in about 30-40 weight percent relative to the amount of deionized water. The free radical polymerization was initiated with commercially available 2,2'-azobis(N,N'-dimethylisobutyramidine) dihydrochloride. About 0.1 mole percent based on the total moles of the monomers of the azo free-radical initiator was employed. The reaction was then degassed by bubbling nitrogen gas through the mixture for 15 minutes. The reaction temperature was maintained between 20-35°C. for 24 hours. The reactions produced transparent or cloudy hard gels of the crosslinked polymers. A large volume of deionized water was added to the polymer product and the polymers were allowed to swell for about 24 hours. The swelled polymers were dried in a forced convection oven at 65.6°C (150°F). The dried polymers were then mechanically blended to a powder.

Some of the polymers were hydrolyzed and neutralized with a strong base such as aqueous sodium hydroxide or aqueous potassium hydroxide. The degree of hydrolysis or neutralization could be controlled by stoichiometrically limiting the amount of base relative to the amount of amide, nitrile, or carboxylic acid functionalities. A suspension of 1 gram of the polymer in about 20 milliliters of 0.5 molar aqueous sodium hydroxide was heated to 95°C. until a light golden-yellow color was obtained. The mixture was then transferred to a dialysis bag with a molecular weight cut-off of 12,000-14,000 and dialyzed exhaustively against distilled water until the viscous polymer gel had reached pH 7. This viscous polymer gel was then poured into a plastic dish and dried in a forced convection oven at 74°C. The dried polymers were then mechanically blended to a powder.

The dried polymers were then tested for deionized water absorption and synthetic urine absorption. About 1 liter of deionized water or synthetic urine was added to 0.1 to 0.5 gram of the dried polymer and allowed to stand for 24 hours. The polymer was then separated from the excess unabsorbed liquid by screening through a stainless steel sieve having openings of 0.149 mm per 2.54 cm (100 mesh per inch). The absorbency was determined by weighing the isolated polymer containing the absorbed liquid and substracting the weight of the dry polymer. The absorbency was measured in units of grams of liquid per grams of polymer. The synthetic urine was prepared by dissolving 0.64 gram CaCl₂, 1.14 gram MgSO₄·7H₂O, 8.20 gram NaCl, and 20.0 gram urea into 1000 gram deionized water. Several of the polymers were tested two or three times, and the experimental error was within plus or minus 2-5 percent. This small experimental error was largely caused by gel blocking and minor diffusion problems that prevented the aqueous liquid from contacting with all the polymer.

**TABLE XVI**

| Control Data | | | | | | | | | |
|---|---|---|---|---|---|---|---|---|---|
| EXP# | AMPS® | AM | AN | AA | X-AA | LINK mole ratio* | XOH | DIW | SU |
| | MOLE PERCENT | | | | | | | ---g/g* -- | |
| 126A | -- | 100 | -- | -- | -- | 0.05 | NO | 17 | 15 |
| 126 | -- | 100 | -- | -- | -- | 0.05 | YES | 1024 | 25 |
| 406R | -- | 100 | -- | -- | -- | 0.05 | YES | 364 | 40 |
| 125A | -- | 100 | -- | -- | -- | 0.20 | NO | 13 | 12.5 |
| 125 | -- | 100 | -- | -- | -- | 0.20 | YES | 295 | 16 |
| 26 | -- | -- | 100 | -- | -- | 0.05 | YES | 608 | 46 |
| 405 | -- | -- | 100 | -- | -- | 0.10 | NO | 0 | 0 |
| 405 | -- | -- | 100 | -- | -- | 0.10 | YES | 414 | 42 |
| 129 | -- | -- | 100 | -- | -- | 0.20 | YES | 352 | 25 |
| 127A | -- | -- | -- | 100 | -- | 0.20 | NO | 21 | 11 |
| 127 | -- | -- | -- | 100 | -- | 0.20 | Neutr. | 423 | 10 |
| 194 | -- | -- | -- | -- | 100 (K) | 0.05 | NO | 669 | 57 |
| 204 | -- | -- | -- | -- | 100 (Na) | 0.05 | NO | 505 | 41 |
| 211 | -- | 13 | -- | -- | 87 | 0.05 | NO | -- | 65 |
| 267 | 3 | 13 | -- | -- | 84 | 0.05 | NO | 350 | 38 |
| 372 | 3 | 20 | -- | -- | 77 | 0.05 | NO | 417 | 47 |
| 20 | 6 | 13 | -- | -- | 81 | 0.05 | NO | 738 | 56 |
| 21 | 6 | 26 | -- | -- | 68 | 0.05 | NO | 533 | 47 |
| 22 | 6 | -- | -- | -- | 94 | 0.05 | NO | 488 | 55 |
| 23 | 10 | 13 | -- | -- | 77 | 0.05 | NO | 570 | 59 |
| 25 | 20 | 13 | -- | -- | 67 | 0.05 | NO | 624 | 62 |
| 19 | 100 | -- | -- | -- | -- | 0.05 | NO | -Soluble- | |
| AMPS ® = 2-acrylamido-2-methylpropane sulfonate (Note: AMPS is a trademark of Lubrizol.) AM = Acrylamide AN = Acrylonitrile AA = Acrylic Acid X-AA = Alkali Salt of Acrylic Acid (Acrylate) LINK = Methylene-bis-acrylamide Crosslinking Agent XOH = Basic Hydrolysis and/or Neutralization with aqueous NaOH or KOH DIW = Deionized Water SU = Synthetic Urine | | | | | | | | | |

| | | | | | | | | | |
|---|---|---|---|---|---|---|---|---|---|
| * mole ratio = moles of crosslinking agent per 100 moles of the ampholytic ion pair and/or comonomer. | | | | | | | | | |
| ** g/g = absorbency units of gram aqueous liquid per gram dried polymer | | | | | | | | | |

### Example II

The control data in Table XVII demonstrates that although commercially available water absorbing materials are highly absorbent to water, they are also dramatically less absorbent to aqueous electrolyte solutions such as salt water and urine. The commercially available water absorbing materials tested include poly(co-acrylamide-co-acrylic acid) grafted onto starch, a commercial acrylamide polymer sold under the trademark "Water Grabber" ® ("Water Grabber" is a trademark of F. P. Products, Inc.), "LUVS" diaper absorbent ("LUVS" is a trademark of Procter & Gamble Co.) , "Pampers" ® diaper absorbent ("Pampers" is a trademark of Procter & Gamble Co.), and "Favor 960" ® (stockhausen, Inc.). The best of these known materials absorb up to about 56 grams of urine per gram of absorbing material, and most of the known polymers absorb much less than 40 grams of urine per gram of absorbing material.

**TABLE XVII**

| Control Data For Commercial Materials | | | |
|---|---|---|---|
| EXP# | Commercial Material | DIW | SU |
| | | -- g/g* -- | |
| 1 | COMMERCIAL STARCH-g-POLY(AM-AA) | 345 | 37 |
| 2 | WATER GRABBER ® (AM COPOLYMER) | 440 | 34 |
| 3 | LUVS ® DIAPER ABSORBENT | 191 | 16 |
| 4 | PAMPERS ® DIAPER ABSORBENT | 171 | 12 |
| 5 | FAVOR 960 ® | 369 | 56 |
| g = graft AM = Acrylamide AA = Acrylic Acid DIW = Deionized Water SU = Synthetic Urine | | | |

| | | | |
|---|---|---|---|
| * g/g = absorbency units of gram aqueous liquid per gram dried polymer | | | |

### Example III

The homopolymers of the ampholytic ion pair monomers with 0.05 weight percent methylene-bis-acrylamide cross linking agent were tested for absorbency to deionized water and synthetic urine according to the method employed in Example I. The absorbency of homopolymers is very poor. See Tables XVIII-XX.

**Table XVIII**

| Control Data For MPDMA/sulfonate Ion Pair Homopolymer | | | | | |
|---|---|---|---|---|---|
| EXP# | MPDMA/AMPS | MPDMA/MES | LINK mole ratio* | DIW | SU |
| | MOLE PERCENT | | | -- g/g** -- | |
| 319 | 100 | -- | 0.05 | 10 | 23 |
| 100 | -- | 100 | 0.05 | 12 | 17 |
| MPDMA/AMPS = 3-methacrylamidopropyldimethylammonium cation 2-acrylamido-2-methylpropane sulfonate anion MPDMA/MES = 3-methacrylamidopropyldimethylammonium cation 2-methacryloyloxyethane sulfonate anion LINK = Methylene-bis-acrylamide Crosslinking Agent DIW = Deionized Water SU = synthetic Urine | | | | | |

| | | | | | |
|---|---|---|---|---|---|
| * mole ratio = moles of crosslinking agent per 100 moles of the ampholytic ion pair and/or comonomer. | | | | | |
| ** g/g = absorbency units of gram aqueous liquid per gram dried polymer | | | | | |

The absorbency to deionized water is about 10 grams water per gram of homopolymer, and only 23 and 17 gram synthetic urine per gram of homopolymer, respectively.

**Table XIX**

| Control Data For MEDMA/sulfonate Ion Pair Homopolymer | | | | |
|---|---|---|---|---|
| MEDMA/AMPS | MEDMA/MES | LINK mole ratio* | DIW | SU |
| ---------MOLE PERCENT -------- | | | -- g/g** -- | |
| 100 | -- | 0.05 | 8 | 10 |
| -- | 100 | 0.05 | 9 | 20 |
| MEDMA/AMPS = 2-methacryloyloxyethyldimethylammonium cation 2-acrylamido-2-methylpropane sulfonate anion MEDMA/MES = 2-methacryloyloxyethyldimethylammonium cation 2-methacryloyloxyethane sulfonate anion LINK = Methylene-bis-acrylamide Cross-Linking Agent DIW = Deionized Water SU = Synthetic Urine | | | | |

| | | | | |
|---|---|---|---|---|
| * mole ratio = mole crosslinking agent per 100 mole of the ampholytic ion pair monomer and the comonomers. | | | | |
| ** g/g = absorbency units of gram aqueous liquid per gram dried polymer | | | | |

The absorbency to deionized water is less than 10 gram water per gram of homopolymer, and only 10 and 20 gram synthetic urine per gram of homopolymer, respectively.

**Table XX**

| Control Data For MEDEA/sulfonate Ion Pair Homopolymer | | | | | |
|---|---|---|---|---|---|
| EXP# | MEDEA/AMPS | MEDEA/MES | LINK MOLE RATIO* | DIW | SU |
| | ----------MOLE PERCENT --- | | | -- g/g** -- | |
| 21 | 100 | -- | 0.05 | 4.6 | 8 |
| 5 | 100 | -- | 0.06 | 7 | 10 |
| 36 | -- | 100 | 0.05 | 13 | 29 |
| MEDEA/AMPS = 2-methacryloyloxyethyldiethylammonium cation 2-acrylamido-2-methylpropane sulfonate anion MEDEA/MES = 2-methacryloyloxyethyldiethylammonium cation 2-methacryloyloxyethane sulfonate anion LINK = Methylene-bis-acrylamide Crosslinking Agent DIW = Deionized Water SU = Synthetic Urine | | | | | |

| | | | | | |
|---|---|---|---|---|---|
| * Mole Ratio is the moles of crosslinking agent per 100 moles of the ion pair plus the moles of any comonomers. | | | | | |
| ** g/g = absorbency units of gram aqueous liquid per gram dried polymer | | | | | |

The absorbency to water is less than 10 gram water per gram of homopolymer, and only 10 and 29 gram synthetic urine per gram of homopolymer, respectively.

### Example IV

The control data in Table XXI demonstrates that although the known ampholytic ion pair 3-methacrylamidopropyltrimethylammonium 2-acrylamido-2-methylpropane sulfonate (MPTMA/AMPS) copolymerized with acrylamide is highly absorbent to deionized water, it is dramatically less absorbent to aqueous electrolyte solutions such as salt water, brine, and urine. The absorbency to synthetic urine is about the same as for the better of the known polymers and commercial materials. The MPTMA/AMPS-acrylamide copolymer also has been grafted onto starch using ceric ion or cobalt-60 irradiation. These starch grafted copolymers are poorly absorbent to deionized water, and only slightly more absorbent to synthetic urine. The better of these known polymers absorbs up to about 56 grams of urine per gram of polymer, but the rest absorb less than 30 grams of urine per gram of polymer.

**Table XXI**

| Control Data For Known MPTMA/AMPS-Acrylamide Copolymers | | | | | | | |
|---|---|---|---|---|---|---|---|
| EXP# | MPTMA/AMPS | AM | LINK mole ratio | Starch | XOH | DIW | SU |
| | MOLE PERCENT | | | | | -- g/g* -- | |
| | 10 | 90 | -- | -- | NO | -soluble- | |
| 87 | 10 | 90 | 0.20 | -- | YES | 428 | 56 |
| ** | 8.56 | 27.30 | -- | 64.86 | NO | 9.83 | 16.21 |
| ** | 8.98 | 41.76 | -- | 49.26 | NO | 11.54 | 16.62 |
| ** | 15.01 | 64.96 | -- | 20.03 | NO | 14.11 | 29.45 |
| MPTMA/AMPS = 3-methacrylamidopropyltrimethylammonium cation/ 2-acrylamido-2-methylpropane sulfonate anion AM = Acrylamide LINK = Methylene-bis-acrylamide Crosslinking Agent XOH = Basic Hydrolysis and/or Neutralization with aqueous NaOH or KOH DIW = Deionized Water SU = Synthetic Urine | | | | | | | |

| | | | | | | | |
|---|---|---|---|---|---|---|---|
| * mole ratio = mole crosslinking agent per 100 mole of the ampholytic ion pair monomer and the comonomers. | | | | | | | |
| ** g/g = absorbency units of gram aqueous liquid per gram dried polymer | | | | | | | |
| *** J. C. Salamone, E. L. Rodriguez, K. C. Lin, L. Quach, A. C. Watterson and I. Ahmed, Polymer 26, 1234-38 (1985). | | | | | | | |

### Example V

The polymers of the present invention formed from components (a), (b) and (c) were prepared according to the method described in Example I, except that the inventive polymers were prepared by mixing the monomers in the proportions given in Tables XXII-XXVII.

The inventive polymers were tested for absorbency to deionized water and synthetic urine. The tested polymers comprise polymers formed by the copolymerization of an effective amount of the components listed in the following tables to produce polymers highly absorbent to aqueous electrolyte solutions.

Some of the inventive polymers in these examples which contain an olefinic comonomer with amide, nitrile, carboxylic acid, or sulfonic acid functionalities or a crosslinking agent with amide, nitrile, carboxylic acid, or sulfonic acid functionalities were hydrolyzed and neutralized with an aqueous base such as aqueous sodium hydroxide or aqueous potassium hydroxide.

**Table XXII**

| Experimental Data For Inventive MPDMA/AMPS Polymers | | | | | | | | | |
|---|---|---|---|---|---|---|---|---|---|
| EXP# | MPDMA/AMPS | AM | AN | AA | X-AA | LINK mole ratio* | XOH | DIW | SU |
| | --------------MOLE PERCENT | | | | | | | -- g/g** -- | |
| 415 | 5 | 95 | -- | -- | -- | 0.05 | YES | 429 | 60 |
| 416 | 10 | 90 | -- | -- | -- | 0.05 | YES | 488 | 71 |
| 417 | 15 | 85 | -- | -- | -- | 0.05 | YES | 516 | 82 |
| 418 | 20 | 80 | -- | -- | -- | 0.05 | YES | 598 | 91 |
| 411 | 5 | -- | 95 | -- | -- | 0.10 | YES | 600 | 98 |
| 412 | 10 | -- | 90 | -- | -- | 0.10 | YES | 551 | 89 |
| 413 | 15 | -- | 85 | -- | -- | 0.10 | YES | 786 | 94 |
| 414 | 20 | -- | 80 | -- | -- | 0.10 | YES | 724 | 117 |
| 309 | 1.4 | 20.3 | -- | -- | 78.3 | 0.05 | NO | 662 | 72 |
| 310 | 2.8 | 20.7 | -- | -- | 76.5 | 0.05 | NO | 674 | 76 |
| 236 | 3 | 13 | -- | -- | 84 | 0.05 | NO | 666 | 72 |
| 232 | 3 | 13 | -- | -- | 84 | 0.05 | NO | 780 | 71 |
| 237 | 6 | 13 | -- | -- | 81 | 0.05 | NO | 700 | 84 |
| 327 | 6 | 20 | -- | -- | 74 | 0.05 | NO | 642 | 80 |
| 233 | 6 | 13 | -- | -- | 81 | 0.05 | NO | 768 | 83 |
| 238 | 10 | 13 | -- | -- | 77 | 0.05 | NO | 782 | 83 |
| 311 | 10 | 20 | -- | -- | 70 | 0.05 | NO | 494 | 70 |
| 239 | 15 | 13 | -- | -- | 72 | 0.05 | NO | 542 | 72 |
| 101 | 1 | -- | -- | -- | 99 | 0.05 | NO | 747 | 90 |
| 102 | 3 | -- | -- | -- | 97 | 0.05 | NO | 832 | 93 |
| 103 | 6 | -- | -- | -- | 94 | 0.05 | NO | 679 | 77 |
| 104 | 10 | -- | -- | -- | 90 | 0.05 | NO | 781 | 79 |
| 105 | 25 | -- | -- | -- | 75 | 0.05 | NO | 620 | 54 |
| MPDMA/AMPS = 3-methacrylamidopropyldimethylammonium cation/ 2-acrylamido-2-methylpropane sulfonate anion AM = Acrylamide AN = Acrylonitrile AA = Acrylic Acid X-AA = Alkali Salt of Acrylic Acid (Acrylate) LINK = Methylene-bis-acrylamide Crosslinking Agent XOH = Basic Hydrolysis and/or Neutralization with aqueous NaOH or KOH DIW = Deionized Water SU = Synthetic Urine | | | | | | | | | |

| | | | | | | | | | |
|---|---|---|---|---|---|---|---|---|---|
| * mole ratio = mole crosslinking agent per 100 moles of the ampholytic ion pair monomer and the comonomer | | | | | | | | | |
| ** g/g = absorbency units of gram aqueous liquid per gram dried polymer | | | | | | | | | |

**Table XXIII**

| Experimental Data For Inventive MPDMA/MES Polymers | | | | | | | | | |
|---|---|---|---|---|---|---|---|---|---|
| EXP# | MPDMA/MES | AM | AN | AA | X-AA | LINK mole ratio* | XOH | DIW | SU |
| | -------------MOLE PERCENT | | | | | | | --g/g** -- | |
| 95 | 1 | -- | -- | -- | 99 | 0.05 | NO | 638 | 88 |
| 96 | 3 | -- | -- | -- | 97 | 0.05 | NO | 645 | 96 |
| 97 | 6 | -- | -- | -- | 94 | 0.05 | NO | 483 | 84 |
| 98 | 10 | -- | -- | -- | 90 | 0.05 | NO | 401 | 73 |
| 99 | 25 | -- | -- | -- | 75 | 0.05 | NO | 386 | 75 |
| MPDMA/AMPS = 3-methacrylamidopropyldimethylammonium cation/ 2-acrylamido-2-methylpropane sulfonate anion AM = Acrylamide AN = Acrylonitrile AA = Acrylic Acid X-AA = Alkali Salt of Acrylic Acid (Acrylate) LINK = Methylene-bis-acrylamide Crosslinking Agent XOH = Basic Hydrolysis and/or Neutralization with aqueous NaOH or KOH DIW = Deionized Water SU = Synthetic Urine | | | | | | | | | |

| | | | | | | | | | |
|---|---|---|---|---|---|---|---|---|---|
| * mole ratio = mole crosslinking agent per 100 moles of the ampholytic ion pair monomer and the comonomer | | | | | | | | | |
| ** g/g = absorbency units of gram aqueous liquid per gram dried polymer | | | | | | | | | |

The data in Tables XXII and XXIII demonstrates that these polymers exhibit significantly improved absorbency to aqueous electrolyte solutions such as urine over the absorbency of the known polymers listed in Table XVI, the commercially available materials listed in Table XVII, the crosslinked MPDMA/sulfonate homopolymers listed in Table XVIII, and the analogous crosslinked MPTMA/AMPS-acrylamide copolymers listed in Table XXI.

The absorbency of these polymers to urine is highly unexpected in view of the fact that the homopolymers of MPDMA/sulfonate with 0.05 weight percent crosslinking agent only absorb about 20 grams of synthetic urine per gram of the polymer. See Table XVIII. This demonstrates that the monomers when combined into the polymers of the present invention act synergistically to increase the absorbency of the polymers to aqueous liquids such as salt water and urine.

Taking an absorbency of about 56 grams of synthetic urine per gram of polymer as about the best of the known polymers, the preferred MPDMA/sulfonate polymers of the present invention exceed this absorbency to urine by 25-63 percent (70-91 grams synthetic urine per gram of inventive polymer, Table XXII, compared to 56 grams urine per gram for the best known materials, Tables XVI, XVII, XVIII and XXI) without sacrificing absorbency to deionized water.

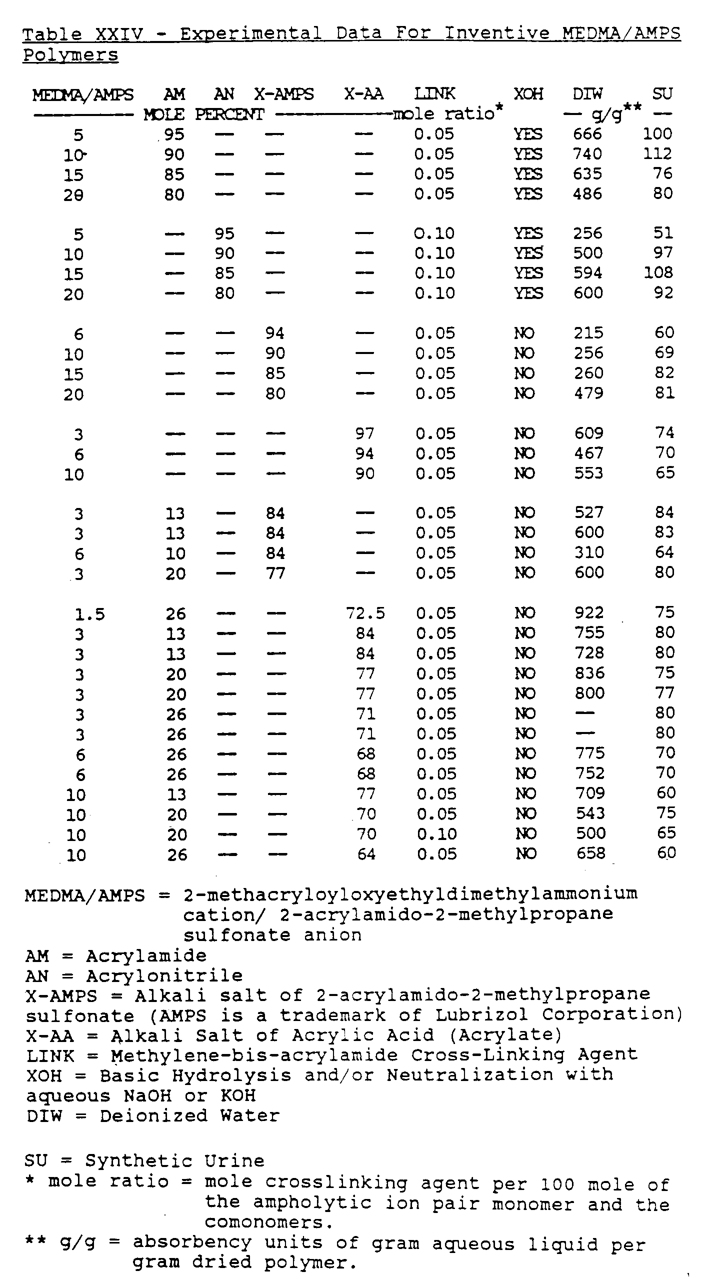

**Table XXV**

| Experimental Data For Inventive MEDMA/MES Polymers | | | | | | | | |
|---|---|---|---|---|---|---|---|---|
| MEDMA/MES | AM | AN | AA | X-AA | LINK mole ratio* | XOH | DIW | SU |
| --------- MOLE PERCENT----------- | | | | | | | --g/g** -- | |
| 10 | 90 | -- | -- | -- | 0.05 | YES | 694 | 106 |
| 14 | 86 | -- | -- | -- | 0.05 | YES | 1200 | 105 |
| 15 | 85 | -- | -- | -- | 0.04 | YES | 1300 | 121 |
| 15 | 85 | -- | -- | -- | 0.05 | YES | 1500 | 120 |
| 20 | 80 | -- | -- | -- | 0.04 | YES | 1200 | 85 |
| 23 | 77 | -- | -- | -- | 0.04 | YES | 1008 | 90 |
| 25 | 75 | -- | -- | -- | 0.04 | YES | 1000 | 90 |
| 50 | 50 | -- | -- | -- | 0.05 | YES | 622 | 100 |
| 6 | -- | 94 | -- | -- | 0.05 | YES | 480 | 70 |
| 10 | -- | 90 | -- | -- | 0.05 | YES | 443 | 77 |
| 15 | -- | 85 | -- | -- | 0.05 | YES | 678 | 110 |
| 20 | -- | 80 | -- | -- | 0.05 | YES | 387 | 106 |
| 25 | -- | 75 | -- | -- | 0.05 | YES | 200 | 70 |
| 50 | -- | 50 | -- | -- | 0.05 | YES | 274 | 45 |
| 3 | -- | -- | -- | 97 | 0.05 | NO | 873 | 70 |
| 3 | 13 | -- | -- | 84 | 0.05 | NO | 616 | 90 |
| 3 | 27 | -- | -- | 70 | 0.05 | NO | 462 | 85 |
| 3 | 35 | -- | -- | 62 | 0.05 | NO | 739 | 80 |
| 3 | 47 | -- | -- | 50 | 0.05 | NO | 694 | 72 |
| 3 | 57 | -- | -- | 40 | 0.05 | NO | 609 | 67 |
| 6 | 13 | -- | -- | 81 | 0.05 | NO | 521 | 83 |
| 10 | 13 | -- | -- | 77 | 0.05 | NO | 358 | 84 |
| 15 | 13 | -- | -- | 72 | 0.05 | NO | 528 | 86 |
| 25 | 13 | -- | -- | 62 | 0.05 | NO | 473 | 74 |
| 10 | 40 | -- | -- | 50 | 0.05 | NO | 739 | 80 |
| MEDMA/MES = 2-methacryloyloxyethyldimethylammonium cation/ 2-methacryloyloxyethane sulfonate anion AM = Acrylamide AN = Acrylonitrile AA = Acrylic Acid X-AA = Alkali Salt of Acrylic Acid (Acrylate) LINK = Methylene-bis-acrylamide Cross-Linking Agent XOH = Basic Hydrolysis and/or Neutralization with aqueous NaOH or KOH DIW = Deionized Water SU = Synthetic Urine | | | | | | | | |

| | | | | | | | | |
|---|---|---|---|---|---|---|---|---|
| * mole ratio = mole crosslinking agent per 100 mole of the ampholytic ion pair monomer and the comonomers. | | | | | | | | |
| ** g/g = absorbency units of gram aqueous liquid per gram dried polymer. | | | | | | | | |

The data in Tables XXIV and XXV demonstrate that these polymers exhibit significantly improved absorbency to aqueous electrolyte solutions such as urine over the absorbency of the known polymers listed in Table XVI, the commercially available materials listed in Table XVII, the crosslinked MEDMA/sulfonate homopolymers listed in Table XIX, and the analogous crosslinked MPTMA/AMPS-acrylamide copolymers listed in Table XXI.

The absorbency of these polymers to urine is highly unexpected in view of the fact that the homopolymers of MEDMA/sulfonate with 0.05 mole percent crosslinking agent only absorb about 10 grams of synthetic urine per gram of the polymer. See Table XIX. This demonstrates that the monomers when combined into the polymers of the present invention act synergistically to increase the absorbency of the polymers to aqueous liquids such as salt water and urine.

Taking an absorbency of about 56 grams of synthetic urine per gram of polymer as about the best of the known polymers, the preferred MEDMA/sulfonate polymers of the present invention exceed this absorbency to urine by 25-116 percent (70-121 grams synthetic urine per gram of inventive polymer, Table XXIV and Table XXV, compared to 56 grams urine per gram for the best known materials, Tables XVI, XVII, XIX and XXI) without sacrificing absorbency to deionized water.

**Table XXVI**

| Experimental Data For Inventive MEDEA/AMPS Polymers | | | | | | | | | |
|---|---|---|---|---|---|---|---|---|---|
| EXP# | MEDEA/AMPS | AM | AN | AA | X-AA | LINK mole ratio* | XOH | DIW | SU |
| | ---------- MOLE PERCENT ----------- | | | | | | | -- g/g** -- | |
| 22 | 5 | 95 | -- | -- | -- | 0.05 | YES | 750 | 102 |
| 23 | 10 | 90 | -- | -- | -- | 0.05 | YES | 750 | 91 |
| 24 | 15 | 85 | -- | -- | -- | 0.05 | YES | 669 | 109 |
| 25 | 20 | 80 | -- | -- | -- | 0.05 | YES | 630 | 116 |
| 6 | 5 | -- | 95 | -- | -- | 0.06 | YES | 507 | 81 |
| 7 | 10 | -- | 90 | -- | -- | 0.06 | YES | 512 | 93 |
| 8 | 15 | -- | 85 | -- | -- | 0.06 | YES | 520 | 99 |
| 9 | 20 | -- | 80 | -- | -- | 0.06 | YES | 567 | 106 |
| 26 | 3 | 20 | -- | -- | 77 | 0.05 | NO | 751 | 100 |
| 27 | 5 | 20 | -- | -- | 75 | 0.05 | NO | 780 | 109 |
| 28 | 10 | 20 | -- | -- | 70 | 0.05 | NO | 744 | 118 |
| MEDEA/AMPS = 2-methacryloyloxyethyldiethylammonium cation/ 2-acrylamido-2-methylpropane sulfonate anion AM = Acrylamide AN = Acrylonitrile AA = Acrylic Acid X-AA = Alkali Salt of Acrylic Acid (Acrylate) LINK = Methylene-bis-acrylamide Cross-Linking Agent XOH = Basic Hydrolysis and/or Neutralization with aqueous NaOH or KOH DIW = Deionized Water SU = Synthetic Urine | | | | | | | | | |

| | | | | | | | | | |
|---|---|---|---|---|---|---|---|---|---|
| * mole ratio = mole crosslinking agent per 100 mole of the ampholytic ion pair monomer and the comonomers. | | | | | | | | | |
| ** g/g = absorbency units of gram aqueous liquid per gram dried polymer | | | | | | | | | |

**Table XXVII**

| Experimental Data For Inventive MEDEA/MES Polymers | | | | | | | | | |
|---|---|---|---|---|---|---|---|---|---|
| EXP# | MEDEA/MES | AM | AN | AA | X-AA | LINK mole ratio* | XOH | DIW | SU |
| | ---------- MOLE PERCENT ----------- | | | | | | | -- g/g** -- | |
| 37 | 6 | 94 | -- | -- | -- | 0.05 | YES | 785 | 100 |
| 165 | 10 | 90 | -- | -- | -- | 0.03 | YES | 1400 | 113 |
| 38 | 10 | 90 | -- | -- | -- | 0.05 | YES | 878 | 110 |
| 166 | 15 | 85 | -- | -- | -- | 0.03 | YES | 1260 | 87 |
| 39 | 15 | 85 | -- | -- | -- | 0.05 | YES | 412 | 101 |
| 167 | 20 | 80 | -- | -- | -- | 0.03 | YES | 966 | 65 |
| 40 | 20 | 80 | -- | -- | -- | 0.05 | YES | 508 | 111 |
| 41 | 25 | 75 | -- | -- | -- | 0.05 | YES | 429 | 94 |
| 42 | 50 | 50 | -- | -- | -- | 0.05 | YES | 389 | 70 |
| 43 | 6 | -- | 94 | -- | -- | 0.05 | YES | 540 | 89 |
| 44 | 10 | -- | 90 | -- | -- | 0.05 | YES | 453 | 110 |
| 45 | 15 | -- | 85 | -- | -- | 0.05 | YES | 502 | 116 |
| 46 | 20 | -- | 80 | -- | -- | 0.05 | YES | 330 | 80 |
| 47 | 25 | -- | 75 | -- | -- | 0.05 | YES | 348 | 75 |
| 48 | 50 | -- | 50 | -- | -- | 0.05 | YES | rnicrogel | |
| 51 | 3 | -- | -- | -- | 97 | 0.05 | NO | 360 | 70 |
| 52 | 3 | 13 | -- | -- | 84 | 0.05 | NO | 629 | 85 |
| 57 | 3 | 20 | -- | -- | 77 | 0.05 | NO | 700 | 81 |
| 58 | 3 | 35 | -- | -- | 62 | 0.05 | NO | 800 | 79 |
| 59 | 3 | 50 | -- | -- | 47 | 0.05 | NO | 757 | 78 |
| 53 | 6 | 13 | -- | -- | 81 | 0.05 | NO | 501 | 109 |
| 54 | 10 | 13 | -- | -- | 77 | 0.05 | NO | 491 | 91 |
| 55 | 20 | 13 | -- | -- | 67 | 0.05 | NO | 500 | 95 |
| 56 | 50 | 13 | -- | -- | 37 | 0.05 | NO | 463 | 76 |
| MEDEA/MES = 2-methacryloyloxyethyldiethylammonium cation/ 2-methacryloyloxyethane sulfonate anion AM = Acrylamide AN = Acrylonitrile AA = Acrylic Acid X-AA = Alkali Salt of Acrylic Acid (Acrylate) LINK = Methylene-bis-acrylamide Cross-Linking Agent XOH = Basic Hydrolysis and/or Neutralization with aqueous NaOH or KOH DIW = Deionized Water SU = Synthetic Urine | | | | | | | | | |

| | | | | | | | | | |
|---|---|---|---|---|---|---|---|---|---|
| * mole ratio = mole crosslinking agent per 100 mole of the ampholytic ion pair monomer and the comonomers. | | | | | | | | | |
| ** g/g = absorbency units of gram aqueous liquid per gram dried polymer | | | | | | | | | |

The data in Tables XXVI and XXVII demonstrate that these polymers exhibit significantly improved absorbency to aqueous electrolyte solutions such as urine over the absorbency of the known polymers listed in Table XVI, the commercially available materials listed in Table XVII, the crosslinked MEDEA/sulfonate homopolymers listed in Table XX, and the analogous crosslinked MPTMA/AMPS-acrylamide copolymers listed in Table XXI.

The absorbency of these polymers to urine is highly unexpected in view of the fact that the homopolymers of MEDEA/sulfonate with 0.05 weight percent crosslinking agent only absorb about 10 grams of synthetic urine per gram of the polymer. See Table XX. This demonstrates that the monomers when combined into the polymers of the present invention act synergistically to increase the absorbency of the polymers to aqueous liquids such as salt water and urine.

Taking an absorbency of about 56 grams of synthetic urine per gram of polymer as about the best of the known polymers, the preferred MEDEA/sulfonate polymers of the present invention exceed this absorbency to urine by 25-107 percent (70-116 grams synthetic urine per gram of inventive polymer, Table XXVI and Table XXVII, compared to 56 grams urine per gram for the best known materials, Tables XVI, XVII, XX, and XXI) without sacrificing absorbency to deionized water.

The improved absorbencies demonstrated in Tables XXII-XXVII translate into large savings in the quantity of polymer required and large savings to the consumer.

### Example VI

The polysaccharide grafted copolymers of the present invention formed from components (a), (b) and (c') in Tables XXVIII-XXXIII were generally prepared according to the following two step procedure.

About 10 grams of reagent grade soluble starch was added to 70 milliliters of deionized water. While stirring under inert nitrogen atmosphere, the soluble starch slurry was heated to 95°C. for 1 hr. after which the heat was removed and the stirred soluble starch slurry was allowed to cool to room temperature, about 22°C. A solution of 0.25 gram ceric ammonium nitrate in 2 milliliters 1 N nitric acid was added to the cooled stirring soluble starch slurry. After about 1 minute, the olefinic comonomer (0.1884 - 0.2547 moles) was then added to the soluble starch slurry mixture. The particular comonomer and relative mole percent added for each of the tested polysaccharide grafted copolymers is provided in the following tables. The mixture was stirred under inert nitrogen atmosphere for two hours.

The mixture was then heated to 60°C., at which point a solution of 0.18 g ceric ammonium nitrate in 1.5 milliliters 1 N nitric acid was added to the mixture. After about 1 minute, a 32 weight percent solution of the ampholytic ion pair monomer dissolved in deionized water was added to the warmed mixture. The particulate monomer and relative mole percent added for each of the tested polysaccharide grafted copolymers is provided in the tables. This new mixture was stirred under nitrogen at 60°C. for another 4 hours.

The pH of the mixture was adjusted to between pH 4 and pH 5. The solid crude polysaccharide grafted copolymer was obtained by evaporating the aqueous solvent in a forced convection oven maintained at 74°C. The crude grafted polymer was washed by boiling in dimethylformamide to remove any non-grafted acrylonitrile homopolymer. It was then thoroughly washed with deionized water to remove any water soluble polymer. The purified grafted material was finally washed with ethanol and dried in a vacuum oven at 60°C. for 24 hours. The dried polymers were then mechanically blended to a powder. The yield of polysaccharide grafted copolymer was typically between 60 and 90 percent based on the total weight of the soluble starch, comonomer, and ampholytic ion pair monomer.

Some of the inventive polysaccharide grafted copolymers containing an olefinic comonomer with amide, nitrile, carboxylic acid, or sulfonic acid functionalities were hydrolyzed and/or neutralized with an aqueous base such as aqueous sodium hydroxide or aqueous potassium hydroxide. The degree of hydrolysis or neutralization could be controlled by stoichiometrically limiting the amount of base relative to the amount of amide, nitrile, or carboxylic acid functionalities. For these examples, a stoichiometric excess of the amount of base was used. A suspension of 1 gram of the polymer in about 20 milliliters of 0.5 molar aqueous sodium hydroxide was heated to 95°C. until a light golden-yellow color was obtained. The mixture was then transferred to a dialysis bag with a molecular weight cut-off of 12,000-14,000 and dialyzed exhaustively against distilled water until the viscous polymer gel had reached pH 7. This viscous polymer gel was then poured into a plastic dish and dried in a forced convection oven at 74°C. The dried polymers were then mechanically blended to a powder.

The polysaccharide grafted copolymers were tested according to the method employed in Example I.

**Table XXVIII**

| Comparative Data with MPDMA/AMPS® Graft Copolymer | | | | | | | |
|---|---|---|---|---|---|---|---|
| Exp. | MPDMA/AMPS® | AMPS® | AN | Yield Weight Percent | XOH | DIW | SU |
| | ----------Mole Percent --------------------------- | | | | | | |
| 3B | -- | -- | 100 | 50 | Yes | 783 | 36 |
| 391 | -- | 10 | 90 | 70 | Yes | 650 | 49 |
| 335 | 19 | -- | 79 | 88 | Yes | 478 | 67 |
| MPDMA/AMPS® = 3-methacrylamidopropyldimethylammonium cation 2-acrylamido-2-methylpropane sulfonate anion AMPS® = 2-acrylamido-2-methylpropane sulfonate (Note: AMPS is a trademark of Lubrizol for 2-acrylamido-2-methylpropane sulfonic acid.) AN = Acrylonitrile XOH = Basic Hydrolysis and/or Neutralization with aqueous NaOH or KOH DIW = Deionized Water SU = Synthetic Urine Dsp = Dispersed | | | | | | | |

**Table XXIX**

| Comparative Data with MPDMA/MES Graft Copolymer | | | | | | | |
|---|---|---|---|---|---|---|---|
| Exp. | MPDMA/MES | MES | AN | Yield Weight Percent | XOH | DIW | SU |
| -------------Mole Percent ------------------------------- | | | | | | | |
| 400 | -- | 10 | 90 | -- | Yes | 798 | 50 |
| 366 | 7 | -- | 93 | 67 | Yes | 556 | 80 |
| 367 | 13 | -- | 87 | 53 | Yes | 550 | 78 |
| MPDMA/MES = 3-methacrylamidopropyldimethylammonium cation 2-methacryloyloxyethane sulfonate anion MES = 2-methacryloyloxyethane sulfonate anion AN = Acrylonitrile XOH = Basic Hydrolysis and/or Neutralization with aqueous NaOH or KOH DIW = Deionized Water SU = Synthetic Urine | | | | | | | |

The data in Tables XXVIII and XXIX demonstrate that these polysaccharide grafted MPDMA/sulfonate copolymers exhibit significantly improved absorbency to aqueous electrolyte solutions such as urine over the absorbency of the known crosslinked polymers listed in Table XVI, the commercially available materials listed in Table XVII, the MPDMA/sulfonate crosslinked homopolymers listed in Table XVIII, and the MPTMA/AMPS-acrylamide copolymers listed in Table XXI.

The absorbency of these polymers to urine is highly unexpected in view of the fact that the homopolymers of MPDMA/sulfonate with 0.05 weight percent cross-linking agent only absorb about 23 grams of synthetic urine per gram of the polymer. See Table XVIII. This demonstrates that the monomers when combined into the polysaccharide grafted MPDMA/sulfonate copolymers of the present invention act synergistically to increase the absorbency of the polymers to aqueous liquids such as salt water and urine.

Taking an absorbency of about 37 grams of synthetic urine per gram of polymer as about the best of the known starch grafted polymers, the preferred polysaccharide grafted MPDMA/sulfonate copolymers of the present invention exceed this absorbency to urine by 81-116 percent (67-80 grams synthetic urine per gram of inventive polysaccharide grafted MPDMA/sulfonate copolymers, Table XXVIII and Table XXIX, compared to 37 grams urine per gram for the best of the known starch grafted polymers Table XVII, and Table XXI without sacrificing absorbency to deionized water.

Taking an absorbency of about 56 grams of synthetic urine per gram of polymer as about the best of the commercially available crosslinked polymers, the preferred polysaccharide grafted MPDMA/sulfonate polymers of the present invention generally exceed this absorbency to urine by 20-43 percent (67-80 grams synthetic urine per gram of inventive polysaccharide grafted MPDMA/sulfonate copolymers, Table XXVIII and Table XXIX, compared to 56 grams urine per gram for the best known materials, Table XVI, Table XVII, Table XVIII, and Table XXI) without sacrificing absorbency to deionized water.

**Table XXX**

| Comparative Data with MEDMA/AMPS® Graft Copolymer | | | | | | | |
|---|---|---|---|---|---|---|---|
| Exp. | MEDMA/AMPS® | AMPS® | AN | Yield Weight Percent | XOH | DIW | SU |
| -------------Mole Percent ------------------------------- | | | | | | | |
| 3B | -- | -- | 100 | 50 | Yes | 783 | 36 |
| 391 | -- | 10 | 90 | 70 | Yes | 650 | 49 |
| 371 | 100 | -- | -- | 44 | | Dsp | Dsp |
| 361 | 7 | -- | 93 | 83 | Yes | 565 | 73 |
| 392 | 10 | -- | 90 | 60 | Yes | 603 | 70 |
| 334 | 20 | -- | 84 | 84 | Yes | 349 | 64 |
| MEDMA/AMPS® = 2-methacryloyloxyethyldimethylammonium cation 2-acrylamido-2-methylpropane sulfonate anion AMPS® = 2-acrylamido-2-methylpropane sulfonate (Note: AMPS is a trademark of Lubrizol for 2-acrylamido-2-methylpropane sulfonic acid.) AN = Acrylonitrile XOH = Basic Hydrolysis and/or Neutralization with aqueous NaOH or KOH DIW = Deionized Water SU = Synthetic Urine Dsp = Dispersed | | | | | | | |

**Table XXXI**

| Comparative Data with MEDMA/MES Graft Copolymer | | | | | | | |
|---|---|---|---|---|---|---|---|
| Exp. | MEDMA/MES | MES | AN | Yield Weight Percent | XOH | DIW | SU |
| -------------Mole Percent ------------------------------- | | | | | | | |
| 400 | -- | 10 | 90 | -- | Yes | 798 | 50 |
| 394 | 10 | -- | 90 | 70 | Yes | 637 | 73 |
| 329 | 20 | -- | 80 | 55 | Yes | 900 | 86 |
| MEDMA/MES = 2-methacryloyloxyethyldimethylammonium cation 2-methacryloyloxyethane sulfonate anion MES = 2-methacryloyloxyethane sulfonate anion AN = Acrylonitrile XOH = Basic Hydrolysis and/or Neutralization with aqueous NaOH or KOH DIW = Deionized Water SU = Synthetic Urine | | | | | | | |

The data in Tables XXX and XXXI demonstrates that these polysaccharide grafted MEDMA/sulfonate copolymers exhibit significantly improved absorbency to aqueous electrolyte solutions such as urine over the absorbency of the known crosslinked polymers listed in Table XVI, the commercially available materials listed in Table XVII, the MEDMA/sulfonate crosslinked homopolymers listed in Table XIX, and the MPTMA/AMPS-acrylamide copolymers listed in Table XXI.

The absorbency of these polymers to urine is highly unexpected in view of the fact that the homopolymers of MEDMA/sulfonate with 0.05 weight percent cross-linking agent only absorb about 10 grams of synthetic urine per gram of the polymer. See Table XIX. This demonstrates that the monomers when combined into the polysaccharide grafted MEDMA/sulfonate copolymers of the present invention act synergistically to increase the absorbency of the polymers to aqueous liquids such as salt water and urine.

Taking an absorbency of about 37 grams of synthetic urine per gram of polymer as about the best of the known starch grafted polymers, the preferred polysaccharide grafted MEDMA/sulfonate copolymers of the present invention exceed this absorbency to urine by 132-232 percent (49-86 grams synthetic urine per gram of inventive polysaccharide grafted MEDMA/sulfonate copolymers, Table XXX and Table XXXI, compared to 37 grams urine per gram for the best of the known starch grafted polymers Table XVII, Table XIX, and Table XXI) without sacrificing absorbency to deionized water. These improved absorbencies translate into large savings in the quantity of grafted polymer required and large savings to the consumer.

Taking an absorbency of about 56 grams of synthetic urine per gram of polymer as about the best of the commercially available crosslinked polymers, the preferred polysaccharide grafted MEDMA/sulfonate polymers of the present invention generally exceed this absorbency to urine by 112-153 percent (64.86 grams synthetic urine per gram of inventive polysaccharide grafted MEDMA/sulfonate copolymers, Table XXX and Table XXXI, compared to 56 grams urine per gram for the best known materials, Table XVI, Table XVII, Table XIX and Table XXI) without sacrificing absorbency to deionized water.

**Table XXXII**

| Comparative Data with MEDEA/AMPS R Graft Copolymer | | | | | | | |
|---|---|---|---|---|---|---|---|
| Exp. | MEDEA/AMPS® | AMPS® | AN | Yield Weight Percent | XOH | DIW | SU |
| -------------Mole Percent ------------------------------- | | | | | | | |
| 3B | -- | -- | 100 | 50 | Yes | 783 | 36 |
| 391 | -- | 10 | 90 | 70 | Yes | 650 | 49 |
| 364 | 6.2 | -- | 93.8 | 73 | Yes | 800 | 70 |
| 365 | 11.7 | -- | 89.3 | 85 | Yes | 585 | 78 |
| MEDEA/AMPS® = 2-methacryloyloxyethyldiethylammonium cation 2-acrylamido-2-methylpropane sulfonate anion AMPS® = 2-acrylamido-2-methylpropane sulfonate (Note: AMPS is a trademark of Lubrizol for 2-acrylamido-2-methylpropane sulfonic acid.) AN = Acrylonitrile XOH = Basic Hydrolysis and/or Neutralization with aqueous NaOH or KOH DIW = Deionized Water SU = Synthetic Urine Dsp = Dispersed | | | | | | | |

**Table XXXIII**

| Comparative Data with MEDEA/MES Graft Copolymer | | | | | | | |
|---|---|---|---|---|---|---|---|
| Exp. | MEDEA/MES | MES | AN | Yield Weight Percent | XOH | DIW | SU |
| -------------Mole Percent ------------------------------- | | | | | | | |
| 400 | -- | 10 | 90 | -- | Yes | 798 | 50 |
| 362 | 6.8 | -- | 93.2 | 58 | Yes | 800 | 70 |
| 363 | 12.7 | -- | 87.3 | 57 | Yes | 616 | 63 |
| MEDEA/MES = 2-methacryloyloxyethyldiethylammonium cation/2-methacryloyloxyethane sulfonate anion MES = 2-methacryloyloxyethane sulfonate anion AN = Acrylonitrile XOH = Basic Hydrolysis and/or Neutralization with aqueous NaOH or KOH DIW = Deionized Water SU = Synthetic Urine | | | | | | | |

The data in Tables XXXII and XXXIII demonstrate that these polysaccharide grafted MEDEA/sulfonate copolymers exhibit significantly improved absorbency to aqueous electrolyte solutions such as urine over the absorbency of the known crosslinked polymers listed in Table XVI, the commercially available materials listed in Table XVII, the MEDEA/sulfonate crosslinked homopolymers listed in Table XX, and the MPTMA/AMPS-acrylamide copolymers listed in Table XXI.

The absorbency of these polymers to urine is highly unexpected in view of the fact that the homopolymers of MEDEA/sulfonate with 0.05 weight percent cross-linking agent only absorb about 8 to 29 grams of synthetic urine per gram of the polymer. See Table XX. This demonstrates that the monomers when combined into the polysaccharide grafted MEDEA/sulfonate copolymers of the present invention act synergistically to increase the absorbency of the polymers to aqueous liquids such as salt water and urine.

Taking an absorbency of about 37 grams of synthetic urine per gram of polymer as about the best of the known starch grafted polymers, the preferred polysaccharide grafted MEDEA/sulfonate copolymers of the present invention exceed this absorbency to urine by 70-110 percent (60-78 grams synthetic urine per gram of inventive polysaccharide grafted MEDEA/sulfonate copolymers, Table XXXII and Table XXXIII, compared to 37 grams urine per gram for the best of the known starch grafted polymers Table XVII, Table XX, and Table XXI) without sacrificing absorbency to deionized water.

Taking an absorbency of about 56 grams of synthetic urine per gram of polymer as about the best of the commercially available crosslinked polymers, the preferred MEDEA/sulfonate polysaccharide grafted polymers of the present invention generally exceed this absorbency to urine by 12-39 percent (63-78 grams synthetic urine per gram of inventive polysaccharide grafted MEDEA/sulfonate copolymers, Table XXXII and Table XXXIII, compared to 56 grams urine per gram for the best known materials, Table XVI, Table XVII, Table XX, and Table XXI) without sacrificing absorbency to deionized water.

The improved absorbencies demonstrated in Tables XXVIII-XXXIII translate into large savings in the quantity of grafted polymer required and large savings to the consumer.

### Example VII

A MEDEA/AMPS ® cotton graft copolymer of the present invention was prepared according to the following procedure.

About 0.23 grams of cotton was soaked with a solution of 34·78·1 aqueous monomer, comonomer and crosslinking agent containing: 2.2161 grams of 34·12·1 potassium acrylate, 0.1293 grams of acrylamide, 0.2966 grams of MEDMA/AMPS ® and 0.0216 grams of TEMED (N,N,N',N',-tetramethylenediamine). The solution was mixed together with 0.228 grams of cotton gauze. Then 0.0038 grams of (NH₄)₂S₂O₈ dissolved in 1.03 grams of water was added to the solution containing the cotton gauze. The polymerization started almost instantaneously. The resultant cotton graft copolymer was then washed with water several times to remove any water soluble components. The cotton graft MEDMA/AMPS ® copolymer appeared to be uniformly coated on the gauze. The graft copolymer was then air dried at room temperature. The cotton graft copolymer retained the soft smooth character of the original cotton gauze. About 0.60 grams of the cotton graft copolymer was then tested for water absorbency. The cotton graft copolymer had a synthetic urine absorbency of about 33 grams of synthetic urine/ gram of cotton graft copolymer. This compares very favorably to cotton gauze which absorbed only 3.7 grams of synthetic urine/gram of cotton gauze.

### Example VIII

The following procedure was used to prepare a polypropylene graft copolymer containing MEDMA/AMPS.

About 0.5 grams of a nonwoven polypropylene fiber mat (denier 1-4 and Phillips Fiber source) was soaked with a solution of 0.0006 grams of MBA, 2.2161 grams of 34·78·1 aqueous potassium acrylate, 0.1293 grams of acrylamide, 0.2966 grams of 34·12·1 aqueous MEDMA/AMPS ® and 0.0216 grams of TEMED. After the mat had soaked for 30 min. a second solution containing 0.0038 grams of (NH₄)₂S₂O₈ in 1.03 grams of water was added to the solution containing the mat. Polymerization immediately began. After 2 hrs. the mat was removed from the solution and washed with water several times. The mat was then air dried at room temperature. The resultant mat retained its soft fiberous nature after the graft polymerization.

About 0.50 grams of grafted polypropylene mat was then test for synthetic urine absorbency. The mat after grafting absorbed 14.4 grams of synthetic urine/gram of mat. This compares quite favorably with the water absorbency of untreated sample of the same untreated polypropylene mat which only absorbed 4.47 grams of synthetic urine/gram of polypropylene mat.

## Claims

1. A polymer suitable for use as an absorbent of an aqueous electrolyte solution, said polymer being formed by copolymerization of the following components:
(a) an ampholytic ion pair monomer comprising
(i) the ammonium cation which is
3-methacrylamidopropyldimethylammonium,
2-methacryloyloxyethyldimethylammonium or
2-methacryloyloxyethyldiethylammonium and
(ii) a sulfonate anion which is 2-acrylamido-2-methylpropane sulfonate, 2-methacryloyloxyethane sulfonate, vinyl sulfonate, styrene sulfonate or a combination of two or more thereof; and
(b) at least one comonomer which is acrylamide, methacrylamide, acrylonitrile, acrylic acid, methacrylic acid, an alkali salt of acrylic acid, an alkali salt of methacrylic acid, 2-acrylamido-2-methylpropane sulfonic acid, an alkali salt of 2-acrylamido-2-methylpropane sulfonic acid, 2-methacryloyloxyethane sulfonic acid, an alkali salt of 2-methacryloyloxyethane sulfonic acid, N-vinyl-2-pyrrolidone, or an amine corresponding to said ammonium cation in (a), namely 3-methacrylamidopropyldimethylamine, 2-methacryloyloxyethyldimethylamine, or 2-methacryloyloxyethyldiethylamine, respectively, or a combination of two or more of said comonomers; and wherein there is further included,
(c) a crosslinking agent which has at least two polymerizable olefinic functionalities wherein each of the olefinic functionalities is suitable for crosslinking with polymer chains formed from components (a) and (b) or
(c') a polymer which is a polysaccharide, polypropylene, or polyethylene onto which the comonomer (b) is graft polymerized and with which there is further graft copolymerized the ampholytic ion pair monomer (a);
wherein the comonomer component (b) and the ion pair monomer component (a) are provided in amounts which are effective to produce a highly absorbent copolymer.

2. A polymer according to claim 1, wherein the crosslinking agent component (c) is N,N-diallylmethacrylamide, diallylamine, N,N-bisacrylamidoacetic acid, N,N'-bisacrylamidoacetic acid methylester, N,N'-methylenebisacrylamide (methylene-bis-acrylamide), N,N-benzylidenebisacrylamide, allylacrylate, diisopropenylbenzene, diallyl succinate, ethylene glycol diacrylate, diallylacrylamide, divinylbenzene or a combination of two or more thereof, in particular wherein the crosslinking agent comprises methylene-bis-acrylamide.

3. A polymer according to claim 1 or 2, wherein the comonomer (b) is acrylamide, acrylonitrile, acrylic acid, an alkali salt of acrylic acid, 2-acrylamido-2-methylpropane sulfonic acid, an alkali salt of 2-acrylamido-2-methyl-propane sulfonic acid or any combination of two or more of said comonomers.

4. A polymer according to any one of the preceding claims, wherein the sulfonate anion is 2-acrylamido-2-methylpropane sulfonate or 2-methacryloyloxyethane sulfonate.

5. A polymer according to any one of the preceding claims, wherein said polymer is formed from components (a), (b) and (c).

6. A polymer according to claim 5, wherein said ammonium cation is 3-methacrylamidopropyldimethylammonium.

7. A polymer according to claim 6, wherein the comonomer is acrylamide and the polymer is formed by the polymerization of the following amounts of components (a), (b) and (c)
| | **range** |
|---|---|
| (a) | 10 - 30 mole-% |
| (b) | 70 - 90 mole-% |
| (c) | 0.01 - 0.3 mole |
wherein (a) plus (b) gives 100 mol-% and the amount of (c) is given as moles per 100 moles of (a) and (b).

8. A polymer according to claim 7, wherein the sulfonate anion is 2-acrylamido-2-methylpropane sulfonate or 2-methacryloyloxyethane sulfonate and the polymer is formed by the polymerization of the following amounts of components (a), (b) and (c)
| | **range** |
|---|---|
| (a) | 10 - 30 mole-% |
| (b) | 70 - 90 mole-% |
| (c) | 0.01 - 0.2 mole |
wherein (a) plus (b) gives 100 mole-% and the amount of (c) is given as moles per 100 moles of (a) and (b).

9. A polymer according to claim 6, wherein the comonomer is acrylonitrile and the polymer is formed by the polymerization of the following amounts of components (a), (b) and (c)
| | **range** |
|---|---|
| (a) | 3 - 35 mole-% |
| (b) | 65 - 97 mole-% |
| (c) | 0.01 - 0.3 mole |
wherein (a) plus (b) gives 100 mole-% and the amount of (c) is given as moles per 100 moles of (a) and (b).

10. A polymer according to claim 9, wherein the sulfonate anion is 2-acrylamido-2-methylpropane sulfonate or 2-methacryloyloxyethane sulfonate and the polymer is formed by the polymerization of the following amounts of components (a), (b) and (c)
| | **range** |
|---|---|
| (a) | 3 - 35 mole-% |
| (b) | 65 - 97 mole-% |
| (c) | 0.01 - 0.2 mole |
wherein (a) plus (b) gives 100 mole-% and the amount of (c) is given as moles per 100 moles of (a) and (b).

11. A polymer according to claim 6, wherein the comonomer is an alkali salt of acrylic acid and the polymer is formed by the polymerization of the following amounts of components (a), (b) and (c)
| | **range** |
|---|---|
| (a) | 1 - 30 mole-% |
| (b) | 70 - 99 mole-% |
| (c) | 0.01 - 0.3 mole |
wherein (a) plus (b) gives 100 mole-% and the amount of (c) is given as moles per 100 moles of (a) and (b)

12. A polymer according to claim 11, wherein the sulfonate anion is 2-acrylamido-2-methylpropane sulfonate and the polymer is formed by the polymerization of the following amounts of components (a), (b) and (c)
| | **range** |
|---|---|
| (a) | 1 - 15 mole-% |
| (b) | 85 - 99 mole-% |
| (c) | 0.01 - 0.2 mole |
wherein (a) plus (b) gives 100 mole-% and the amount of (c) is given as moles per 100 moles of (a) and (b)

13. A polymer according to claim 11, wherein the sulfonate anion is 2-methacryloyloxyethane sulfonate and the polymer is formed by the polymerization of the following amounts of components (a), (b) and (c)
| | **range** |
|---|---|
| (a) | 1 - 25 mole-% |
| (b) | 75 - 99 mole-% |
| (c) | 0.01 - 0.2 mole |
wherein (a) plus (b) gives 100 mole-% and the amount of (c) is given as moles per 100 moles of (a) and (b).

14. A polymer according to claim 6, wherein the comonomer is a combination of acrylamide and an alkali salt of acrylic acid and the polymer is formed by the polymerization of the following amounts of components (a), (b) and (c)
| | **range** |
|---|---|
| (a) | 1 - 20 mole-% |
| (b) | 10 - 25 mole-% of acrylamide |
| | and |
| | 55 - 89 mole-% of alkali salt of acrylic acid |
| (c) | 0.01 - 0.3 mole |
wherein (a) plus (b) gives 100 mole-% and the amount of (c) is given as moles per 100 moles of (a) and (b).

15. A polymer according to claim 14, wherein the sulfonate anion is 2-acrylamido-2-methylpropane sulfonate or 2-methacryloyloxyethane sulfonate and the polymer is formed by the polymerization of the following amounts of components (a), (b) and (c)
| | **range** |
|---|---|
| (a) | 1.5 - 15 mole-% |
| (b) | 10 - 21 mole-% of acrylamide |
| | and |
| | 64 - 88.5 mole-% of alkali salt of acrylic acid |
| (c) | 0.01 - 0.2 mole |
wherein (a) plus (b) gives 100 mole-% and the amount of (c) is given as moles per 100 moles of (a) and (b).

16. A polymer according to claim 5, wherein said ammonium cation is 2-methacryloyloxyethyldimethylammonium.

17. A polymer according to claim 16, wherein the comonomer is acrylamide and the polymer is formed by the polymerization of the following amounts of components (a), (b) and (c)
| | **range** |
|---|---|
| (a) | 3 - 50 mole-% |
| (b) | 50 - 97 mole-% |
| (c) | 0.01 - 0.3 mole |
wherein (a) plus (b) gives 100 mole-% and the amount of (c) is given as moles per 100 moles of (a) and (b).

18. A polymer according to claim 17, wherein the sulfonate anion is 2-acrylamido-2-methylpropane sulfonate and the polymer is formed by the polymerization of the following amounts of components (a), (b) and (c)
| | **range** |
|---|---|
| (a) | 3 - 20 mole-% |
| (b) | 97 - 80 mole-% |
| (c) | 0.01 - 0.2 mole |
wherein (a) plus (b) gives 100 mole-% and the amount of (c) is given as moles per 100 moles of (a) and (b).

19. A polymer according to claim 17, wherein the sulfonate anion is 2-methacryloyloxyethane sulfonate and the polymer is formed by the polymerization of the following amounts of components (a), (b) and (c)
| | **range** |
|---|---|
| (a) | 10 - 50 mole-% |
| (b) | 50 - 90 mole-% |
| (c) | 0.01 - 0.2 mole |
wherein (a) plus (b) gives 100 mole-% and the amount of (c) is given as moles per 100 moles of (a) and (b).

20. A polymer according to claim 16, wherein the comonomer is acrylonitrile and the polymer is formed by the polymerization of the following amounts of components (a), (b) and (c)
| | **range** |
|---|---|
| (a) | 6 - 25 mole-% |
| (b) | 75 - 94 mole-% |
| (c) | 0.01 - 0.3 mole |
wherein (a) plus (b) gives 100 mole-% and the amount of (c) is given as moles per 100 moles of (a) and (b).

21. A polymer according to claim 20, wherein the sulfonate anion is 2-acrylamido-2-methylpropane sulfonate and the polymer is formed by the polymerization of the following amounts of components (a), (b) and (c)
| | **range** |
|---|---|
| (a) | 10 - 20 mole-% |
| (b) | 90 - 80 mole-% |
| (c) | 0.01 - 0.1 mole |
wherein (a) plus (b) gives 100 mole-% and the amount of (c) is given as moles per 100 moles of (a) and (b).

22. A polymer according to claim 20, wherein the sulfonate anion is 2-methacryloyloxyethane sulfonate and the polymer is formed by the polymerization of the following amounts of components (a), (b) and (c)
| | **range** |
|---|---|
| (a) | 15 - 20 mole-% |
| (b) | 80 - 85 mole-% |
| (c) | 0.01 - 0.2 mole |
wherein (a) plus (b) gives 100 mole-% and the amount of (c) is given as moles per 100 moles of (a) and (b).

23. A polymer according to claim 16, wherein the comonomer is an alkali salt of acrylic acid and the polymer is formed by the polymerization of the following amounts of components (a), (b) and (c)
| | **range** |
|---|---|
| (a) | 3 - 6 mole-% |
| (b) | 94 - 97 mole-% |
| (c) | 0.01 - 0.3 mole |
wherein (a) plus (b) gives 100 mole-% and the amount of (c) is given as moles per 100 moles of (a) and (b).

24. A polymer according to claim 23, wherein the sulfonate anion is 2-acrylamido-2-methylpropane sulfonate and the polymer is formed by the polymerization of the following amounts of components (a), (b) and (c)
| | **range** |
|---|---|
| (a) | about 3 mole-% |
| (b) | about 97 mole-% |
| (c) | 0.01 - 0.1 mole |
wherein (a) plus (b) gives 100 mole-% and the amount of (c) is given as moles per 100 moles of (a) and (b).

25. A polymer according to claim 23, wherein the sulfonate anion is 2-methacryloyloxyethane sulfonate and the polymer is formed by the polymerization of the following amounts of components (a), (b)and (c)
| | **range** |
|---|---|
| (a) | about 3 mole-% |
| (b) | about 97 mole-% |
| (c) | 0.01 - 0.2 mole |
wherein (a) plus (b) gives 100 mole-% and the amount of (c) is given as moles per 100 moles of (a) and (b).

26. A polymer according to claim 16, wherein the comonomer is an alkali salt of 2-acrylamido-2-methylpropane sulfonic acid and the polymer is formed by the polymerization of the following amounts of components (a), (b) and (c)
| | **range** |
|---|---|
| (a) | 10 - 25 mole-% |
| (b) | 75 - 90 mole-% |
| (c) | 0.01 - 0.3 mole |
wherein (a) plus (b) gives 100 mole-% and the amount of (c) is given as moles per 100 moles of (a) and (b).

27. A polymer according to claim 26, wherein the sulfonate anion is 2-acrylamido-2-methylpropane sulfonate or 2-methacryloyloxyethane sulfonate and the polymer is formed by the polymerization of the following amounts of components (a), (b) and (c)
| | **range** |
|---|---|
| (a) | 10 - 25 mole-% |
| (b) | 75 - 90 mole-% |
| (c) | 0.01 - 0.2 mole |
wherein (a) plus (b) gives 100 mole-% and the amount of (c) is given as moles per 100 moles of (a) and (b).

28. A polymer according to claim 16, wherein the comonomer is a combination of acrylamide and an alkali salt of 2-acrylamido-2-methylpropane sulfonic acid and the polymer is formed by the polymerization of the following amounts of components (a), (b) and (c)
| | **range** |
|---|---|
| (a) | 1 - 10 mole-% |
| (b) | 13 - 25 mole-% of acrylamide |
| | and |
| | 65 - 86 mole-% of alkali salt of 2-acrylamide-2-methylpropane sulfonic acid |
| (c) | 0.01 - 0.2 mole |
wherein (a) plus (b) gives 100 mole-% and the amount of (c) is given as moles per 100 moles of (a) and (b).

29. A polymer according to claim 28, wherein the sulfonate anion is 2-acrylamide-2-methylpropane sulfonate or 2-methacryloyloxyethane sulfonate and the polymer is formed by the polymerization of the following amounts of components (a), (b) and (c)
| | **range** |
|---|---|
| (a) | 1 - 10 mole-% |
| (b) | 13 - 25 mole-% of acrylamide |
| | and |
| | 65 - 86 mole-% of alkali salt of 2-acrylamide-2-methylpropane sulfonic acid |
| (c) | 0.01 - 0.2 mole |
wherein (a) plus (b) gives 100 mole-% and the amount of (c) is given as moles per 100 moles of (a) and (b).

30. A polymer according to claim 16, wherein the comonomer is a combination of acrylamide and an alkali salt of acrylic acid and the polymer is formed by the polymerization of the following amounts of components (a), (b) and (c)
| | **range** |
|---|---|
| (a) | 1 - 30 mole-% |
| (b) | 10 - 50 mole-% of acrylamide |
| | and |
| | 20 - 89 mole-% of alkali salt of acrylic acid |
| (c) | 0.01 - 0.3 mole |
wherein (a) plus (b) gives 100 mole-% and the amount of (c) is given as moles per 100 moles of (a) and (b).

31. A polymer according to claim 30, wherein the sulfonate anion is 2-acrylamido-2-methylpropane sulfonate and the polymer is formed by the polymerization of the following amounts of components (a), (b) and (c)
| | **range** |
|---|---|
| (a) | 1 - 6 mole-% |
| (b) | 13 - 26 mole-% of acrylamide |
| | and |
| | 68 - 86 mole-% of alkali salt of acrylic acid |
| (c) | 0.01 - 0.2 mole |
wherein (a) plus (b) gives 100 mole-% and the amount of (c) is given as moles per 100 moles of (a) and (b).

32. A polymer according to claim 30, wherein the sulfonate anion is 2-methacryloyloxyethane sulfonate and the polymer is formed by the polymerization of the following amounts of components (a), (b) and (c)
| | **range** |
|---|---|
| (a) | 3 - 25 mole-% |
| (b) | 13 - 47 mole-% of acrylamide |
| | and |
| | 28 - 84 mole-% of alkali salt of acrylic add |
| (c) | 0.01 - 02 mole |
wherein (a) plus (b) gives 100 mole-% and the amount of (c) is given as moles per 100 moles of (a) and (b).

33. A polymer according to claim 5, wherein said ammonium cation is 2-methacryloyloxyethyldiethylammonium.

34. A polymer according to claim 33, wherein the comonomer is acrylamide and the polymer is formed by the polymerization of the following amounts of components (a), (b) and (c)
| | **range** | **preferred range** |
|---|---|---|
| (a) | 3 - 50 mole-% | 3 - 30 mole-% |
| (b) | 50 - 97 mole-% | 70 - 97 mole-% |
| (c) | 0.01 - 0.3 mole | 0.03 - 0.2 mole |
wherein (a) plus (b) gives 100 mole-% and the amount of (c) is given as moles per 100 moles of (a) and (b).

35. A polymer according to claim 34, wherein the sulfonate anion is 2-acrylamido-2-methylpropane sulfonate and the polymer is formed by the polymerization of the following amounts of components (a), (b) and (c)
| | **range** |
|---|---|
| (a) | 3 - 30 mole-% |
| (b) | 70 - 97 mole-% |
| (c) | 0.01 - 0.2 mole |
wherein (a) plus (b) gives 100 mole-% and the amount of (c) is given as moles per 100 moles of (a) and (b).

36. A polymer according to claim 34, wherein the sulfonate anion is 2-methacryloyloxyethane sulfonate and the polymer is formed by the polymerization of the following amounts of components (a), (b) and (c)
| | **range** |
|---|---|
| (a) | 3 - 50 mole-% |
| (b) | 50 - 97 mole-% |
| (c) | 0.01 - 0.2 mole |
wherein (a) plus (b) gives 100 mole-% and the amount of (c) is given as moles per 100 moles of (a) and (b).

37. A polymer according to claim 33, wherein the comonomer is acrylonitrile and the polymer is formed by the polymerization of the following amounts of components (a), (b) and (c)
| | **range** |
|---|---|
| (a) | 3 - 30 mole-% |
| (b) | 70 - 97 mole-% |
| (c) | 0.01 - 0.3 mole |
wherein (a) plus (b) gives 100 mole-% and the amount of (c) is given as moles per 100 moles of (a) and (b).

38. A polymer according to claim 37, wherein the sulfonate anion is 2-acrylamido-2-methylpropane sulfonate and the polymer is formed by the polymerization of the following amounts of components (a), (b) and (c)
| | **range** |
|---|---|
| (a) | 3 - 25 mole-% |
| (b) | 75 - 97 mole-% |
| (c) | 0.01 - 0.2 mole |
wherein (a) plus (b) gives 100 mole-% and the amount of (c) is given as moles per 100 moles of (a) and (b).

39. A polymer according to claim 37, wherein the sulfonate anion is 2-methacryloyloxyethane sulfonate and the polymer is formed by the polymerization of the following amounts of components (a), (b) and (c)
| | **range** |
|---|---|
| (a) | 3 - 30 mole-% |
| (b) | 70 - 97 mole-% |
| (c) | 0.01 - 0.2 mole |
wherein (a) plus (b) gives 100 mole-% and the amount of (c) is given as moles per 100 moles of (a) and (b).

40. A polymer according to claim 33, wherein the comonomer is an alkali salt of acrylic acid and the polymer is formed by the polymerization of the following amounts of components (a), (b) and (c)
| | **range** |
|---|---|
| (a) | 3 - 6 mole-% |
| (b) | 94 - 97 mole-% |
| (c) | 0.01 - 0.3 mole |
wherein (a) plus (b) gives 100 mole-% and the amount of (c) is given as moles per 100 moles of (a) and (b).

41. A polymer according to claim 40, wherein the sulfonate anion is 2-acrylamido-2-methylpropane sulfonate or 2-methacryloyloxyethane sulfonate and the polymer is formed by the polymerization of the following amounts of components (a), (b) and (c)
| | **range** |
|---|---|
| (a) | about 3 mole-% |
| (b) | about 97 mole-% |
| (c) | 0.03 - 0.2 mole |
wherein (a) plus (b) gives 100 mole-% and the amount of (c) is given as moles per 100 moles of (a) and (b).

42. A polymer according to claim 33, wherein the comonomer is a combination of acrylamide and alkali salt of acrylic acid and the polymer is formed by the polymerization of the following amounts of components (a), (b) and (c)
| | **range** |
|---|---|
| (a) | 1 - 55 mole-% |
| (b) | 10 - 55 mole-% of acrylamide |
| | and |
| | 32 - 89 mole-% of alkali salt of acrylic acid |
| (c) | 0.01 - 0.3 mole |
wherein (a) plus (b) gives 100 mole-% and the amount of (c) is given as moles per 100 moles of (a) and (b).

43. A polymer according to claim 42, wherein the sulfonate anion is 2-acrylamido-2-methylpropane sulfonate and the polymer is formed by the polymerization of the following amounts of components (a), (b) and (c)
| | **range** |
|---|---|
| (a) | 3 - 15 mole-% |
| (b) | 15 - 30 mole-% of acrylamide |
| | and |
| | 55 - 82 mole-% of alkali salt of acrylic acid |
| (c) | 0.01 - 0.2 mole |
wherein (a) plus (b) gives 100 mole-% and the amount of (c) is given as moles per 100 moles of (a) and (b).

44. A polymer according to claim 42, wherein the sulfonate anion is 2-methacryloyloxyethane sulfonate and the polymer is formed by the polymerization of the following amounts of components (a), (b) and (c)
| | **range** |
|---|---|
| (a) | 1 - 55 mole-% |
| (b) | 10 - 55 mole-% of acrylamide |
| | and |
| | 32 - 89 mole-% of alkali salt of acrylic acid |
| (c) | 0.01 - 0.2 mole |
wherein (a) plus (b) gives 100 mole-% and the amount of (c) is given as moles per 100 moles of (a) and (b).

45. A polymer according to any one of claim 1-4, wherein said polymer is formed from components (a), (b) and (c').

46. A polymer according to claim 45, wherein in during the graft polymerization there is also copolymerized a cross-linking agent.

47. A polymer according to claim 45, wherein said component (c') is a polysaccharide which is a soluble starch, cellulose, glycogen or any combination of two or more thereof or polypropylene, in particular wherein said component (c') is polypropylene or a polysaccharide which is a soluble starch or cellulose; and the comonomer is acrylamide, acrylonitrile or an alkali salt of acrylic acid.

48. A polymer according to claim 47, wherein said ammonium cation is 3-methacrylamidopropyldimethylammonium (MPDMA) and said polymer comprises 1 to 50 weight-% of said component (c') and 50 to 99 weight-% of the comonomer and ampholytic ion pair grafted therewith, wherein the amounts of comonomer and ampholytic ion pair are within one of the following sets of ranges given in mole-%:
| **Ion Pair** | **Comonomer** | | |
|---|---|---|---|
| **MPDMA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80 |
wherein AM is acrylamide, AN is acrylonitrile and X-AA is alkali salt of acrylic acid.

49. A polymer according to claim 47, wherein said ammonium cation is 3-methacrylamidopropyldimethylammonium (MPDMA) and said polymer comprises 5 to 30 weight-% of component (c') and 70 to 95 weight-% of the comonomer and ampholytic ion pair grafted therewith, wherein the amounts of comonomer and ampholytic ion pair are within one of the following sets of ranges given in mole-%:
| **Ion Pair** | | **Comonomer** | | |
|---|---|---|---|---|
| **MPDMA/AMPS** | **MPDMA/MES** | **AM** | **AN** | **X-AA** |
| 5 - 25 | - | - | 75 - 95 | - |
| - | 5 - 20 | - | 80 - 95 | - |
| 5 - 20 | - | 95 - 80 | - | - |
| - | 10 - 20 | 90 - 80 | - | - |
| 3 - 15 | - | - | - | 97 - 85 |
| - | 3 - 10 | - | - | 97 - 90 |
wherein AMPS is 2-acrylamido-2-methylpropane sulfonate, MES is 2-methacryloyloxyethane sulfonate, AM is acrylamide, AN is acrylonitrile and X-AA is alkali salt of acrylic acid.

50. A polymer according to claim 47, wherein said ammonium cation is 3-methacryloyloxyethyldimethylammonium (MEDMA) and said polymer comprises 1 to 50 weight percent of said component (c') and 50 to 99 weight percent of the comonomer and ampholytic ion pair grafted therewith, wherein the amounts of comonomer and ampholytic ion pair are within one of the following sets of ranges given in mole-%:
| **Ion Pair** | **Comonomer** | | |
|---|---|---|---|
| **MEDMA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80 |
wherein AM is acrylamide, AN is acrylonitrile and X-AA is alkali salt of acrylic acid.

51. A polymer according to claim 47, wherein said ammonium cation is 2-methacryloyloxyethyldimethylammonium (MEDMA) and said polymer comprises 5 to 30 weight-% of component (c') and 70 to 95 weight-% of the comonomer and ampholytic ion pair grafted therewith, wherein the amounts of comonomer and ampholytic ion pair are within one of the following sets of ranges given in mole-%:
| **Ion Pair** | | **Comonomer** | | |
|---|---|---|---|---|
| **MEDMA/AMPS** | **MEDMA/MES** | **AM** | **AN** | **X-AA** |
| 7 - 20 | - | - | 93 - 80 | - |
| - | 10 - 25 | - | 75 - 90 | - |
| 5 - 15 | - | 95 - 85 | - | - |
| - | 10 - 15 | 90 - 85 | - | - |
| 3 - 15 | - | - | - | 97 - 85 |
| - | 3 - 15 | - | - | 97 - 85 |
wherein AMPS is 2-acrylamido-2-methylpropane sulfonate, MES is 2-methacryloyxloxyethane sulfonate, AM is acrylamide, AN is acrylonitrite and X-AA is alkali salt of acrylic acid.

52. A polymer according to claim 47, wherein said ammonium cation is 3-methacryloyloxyethyldiethylammonium (MEDEA) and said polymer comprises 1 to 50 weight percent of said component (c') and 50 to 99 weight-% of the comonomer and amphoytic ion pair grafted therewith, wherein the amounts of comonomer and ampholytic ion pair are within one of the following sets of ranges given in mole-%:
| **Ion Pair** | **Comonomer** | | |
|---|---|---|---|
| **MEDEA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80 |
wherein AM is acrylamide, AN is acrylonitrile and X-AA is alkali salt of acrylic acid.

53. A polymer according to claim 47, wherein said ammonium cation is 3-methacryloyloxyethyldiethylammonium (MEDEA) and said polymer comprises 5 to 30 weight-% of component (c') and 70 to 95 weight-% of the comonomer and ampholytic ion pair grafted therewith, wherein the amounts of comonomer and ampholytic ion pair are within one of the following sets of ranges given in mole-%:
| **Ion Pair** | | **Comonomer** | | |
|---|---|---|---|---|
| **MEDEA/AMPS** | **MEDEA/MES** | **AM** | **AN** | **X-AA** |
| 5 - 20 | - | - | 95 - 80 | - |
| - | 5 - 20 | - | 95 - 80 | - |
| 5 - 15 | - | 95 - 85 | - | - |
| - | 10 - 15 | 90 - 85 | - | - |
| 3 - 15 | - | - | - | 97 - 85 |
| - | 3 - 10 | - | - | 97 - 90 |
wherein AMPS is 2-acrylamido-2-methylpropane sulfonate, MES is 2-methacryloyloxyethane sulfonate, AM is acrylamide, AN is acrylonitrile and X-AA is alkali salt of acrylic acid.

54. A polymer according to any one of the preceding claims, wherein the polymer is at least partially hydrolyzed or wherein the polymer is at least partially neutralized.

55. A paper towel containing therein the polymer of any one of the preceding claims.

56. A disposable diaper containing therein the polymer of any one of claims 1-54.

57. A method of absorbing aqueous electrolyte solutions comprising contacting the polymer of any one of claims 1-54, the paper towel of claim 55 or the disposable diaper of claim 56 with an aqueous electrolyte solution, in particular wherein the aqueous electrolyte solution is tap water, salt water, brine or urine.

58. A process to produce a polymer by copolymerization of the following components:
(a) an ampholytic ion pair monomer comprising
(i) the ammonium cation which is
3-methacrylamidopropyldimethylammonium,
2-methacryloyloxyethyldimethylammonium or
2-methacryloyloxyethyldiethylammonium and
(ii) a sulfonate anion which is 2-acrylamido-2-methylpropane sulfonate, 2-methacryloyloxyethane sulfonate, vinyl sulfonate, styrene sulfonate or a combination of two or more thereof; and
(b) at least one comonomer which is acrylamide, methacrylamide, acrylonitrile, acrylic acid, methacrylic acid, an alkali salt of acrylic acid, an alkali salt of methacrylic acid, 2-acrylamido-2-methylpropane sulfonic acid, an alkali salt of 2-acrylamido-2-methylpropane sulfonic acid, 2-methacryloyloxyethane sulfonic acid, an alkali salt of 2-methacryloyloxyethane sulfonic add, N-vinyl-2-pyrrolidone, or an amine corresponding to said ammonium cation in (a), namely 3-methacrylamidopropyldimethylamine, 2-methacryloyloxyethyldimethylammonium, or 2-methacryloyloxyethyldiethylamine, respectively, or a combination of two or more of said comonomers; and wherein there is further included,
(c) a crosslinking agent which has at least two polymerizable olefinic functionalities wherein each of the olefinic functionalities is suitable for crosslinking with polymer chains formed from components (a) and (b) or
(c') a polymer which is a polysaccharide, polypropylene, or polyethylene onto which the comonomer (b) is graft polymerized and with which there is further graft copolymerized the ampholytic ion pair monomer (a);
wherein the comonomer component (b) and the ion pair monomer component (a) are provided in amounts which are effective to produce a highly absorbent copolymer.

## Patentansprüche

1. Zur Verwendung als Absorptionsmittel für eine wäßrige Flüssigkeit geeignetes Polymeres, das durch Polymerisation der folgenden Komponenten gebildet wird:
(a) ein ampholytisches Ionenpaar-Monomeres, umfassend
(i) das Ammoniumkation 3-Methacrylamidopropyldimethylammonium, 2-Methacryloyloxyethyldimethylammonium oder 2 -Methacryloyloxyethyldiethylammonium und
(ii) ein Sulfonatanion, bei dem es sich um 2-Acrylamido-2-methylpropansulfonat, 2-Methacryloyloxyethansulfonat, Vinylsulfonat, Styrolsulfonat oder um eine Kombination aus zwei oder mehr dieser Bestandteile handelt; und
(b) mindestens ein Comonomeres, bei dem es sich handelt um Acrylamid, Methacrylamid, Acrylnitril, Acrylsäure, Methacrylsäure, ein Alkalisalz von Acrylsäure, ein Alkalisalz von Methacrylsäure, 2-Acrylamido-2-methylpropansulfonsäure, ein Alkalisalz von 2-Acrylamido-2-methylpropansulfonsaure, 2-Methacryloyloxyethansulfonsäure, ein Alkalisalz von 2-Methacryloyloxyethansulfonsäure, N-Vinyl-2-pyrrolidon oder ein Amin entsprechend dem Ammoniumkation (a) (i), nämlich 3-Methacrylamidopropyldimethylamin, 2-Methacryloyloxyethyldimethylamin bzw. 2-Methacryloyloxyethyldiethylamin oder eine Kombination von zwei oder mehr dieser Comonomeren;
und wobei ferner enthalten ist:
(c) ein Vernetzungsmittel, das mindestens zwei polymerisierbare olefinische Funktionalitäten aufweist, wobei sich die einzelnen olefinischen Funktionalitäten zur Vernetzung mit aus den Komponenten (a) und (b) gebildeten Polymerketten eignen, oder
(c') ein Polymeres, bei dem es sich um ein Polysaccharid, Polypropylen oder Polyethylen handelt, auf das das Comonomere (b) pfropfpolymerisiert ist und das ferner mit dem ampholytischen Ionenpaar-Monomeren (a) pfropfcopolymerisiert ist;
wobei die Comonomerkomponente (b) und die Ionenpaar-Monomerkomponente (a) in Mengen bereitgestellt werden, die zur Bildung eines stark absorbierenden Copolymeren befähigt sind.

2. Polymeres nach Anspruch 1, wobei es sich bei der Vernetzungsmittelkomponente (c) handelt um N,N-Diallylmethacrylamid, Diallylamin, N,N-Bisacrylamidoessigsäure, N,N'-Bisacrylamidoessigäuremethylester, N,N'-Methylenbisacrylamid (Methylen-bis-acrylamid), N,N-Benzylidenbisacrylamid, Allylacrylat, Diisopropenylbenzol, Diallylsuccinat, Ethylenglykoldiacrylat, Diallylacrylamid, Divinylbenzol oder eine Kombination von zwei oder mehr dieser Bestandteile, wobei es sich beim Vernetzungsmittel insbesondere um Methylen-bis-acrylamid handelt.

3. Polymeres nach Anspruch 1 oder 2, wobei es sich beim Comonomeren (b) handelt um Acrylamid, Acrylnitril, Acrylsäure, ein Alkalisalz von Acrylsäure, 2-Acrylamido-2-methylpropansulfonsäure, ein Alkalisalz von 2-Acrylamido-2-methylpropansulfonsäure oder eine beliebige Kombination von zwei oder mehr dieser Comonomeren.

4. Polymeres nach einem der vorstehenden Ansprüche, wobei es sich beim Sulfonat-Anion um 2-Acrylamido-2-methylpropansulfonat oder 2-Methacryloyloxyethansulfonat handelt.

5. Polymeres nach einem der vorstehenden Ansprüche, wobei das Polymere aus den Komponenten (a), (b) und (c) gebildet ist.

6. Polymeres nach Anspruch 5, wobei es sich beim Ammoniumkation um 3-Methacrylamidopropyldimethylammonium handelt.

7. Polymeres nach Anspruch 6, wobei es sich beim Comonomeren um Acrylamid handelt und das Polymere durch Polymerisation der folgenden Mengen der Komponenten (a), (b) und (c) gebildet worden ist:
| | **Bereich** |
|---|---|
| (a) | 10 - 30 Mol-% |
| (b) | 70 - 90 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

8. Polymeres nach Anspruch 7, wobei es sich beim Sulfonat-Anion um 2-Acrylamido-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 10 - 30 Mol-% |
| (b) | 70 - 90 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

9. Polymeres nach Anspruch 6, wobei es sich beim Comonomeren um Acrylnitril handelt und das Polymere durch Polymerisation der folgenden Mengen der Komponenten (a), (b) und (c) gebildet worden ist:
| | **Bereich** |
|---|---|
| (a) | 3 - 35 Mol-% |
| (b) | 65 - 97 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

10. Polymeres nach Anspruch 9, wobei es sich beim Sulfonat-Anion um 2-Acrylamido-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 3 - 35 Mol-% |
| (b) | 65 - 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

11. Polymeres nach Anspruch 6, wobei es sich beim Comonomeren um ein Alkalisalz von Acrylsäure handelt und das Polymere durch Polymerisation der folgenden Mengen der Komponenten (a), (b) und (c) gebildet worden ist:
| | **Bereich** |
|---|---|
| (a) | 1 - 30 Mol-% |
| (b) | 70 - 99 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

12. Polymeres nach Anspruch 11, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 1 - 15 Mol-% |
| (b) | 85 - 99 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

13. Polymeres nach Anspruch 11, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 1 - 25 Mol-% |
| (b) | 75 - 99 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

14. Polymeres nach Anspruch 6, wobei es sich beim Comonomeren um eine Kombination von Acrylamid und einem Alkalisalz von Acrylsäure handelt und das Polymere durch Polymerisation der folgenden Mengen der Komponenten (a), (b) und (c) gebildet worden ist:
| | **Bereich** |
|---|---|
| (a) | 1 - 20 Mol-% |
| (b) | 10 - 25 Mol-% Acrylamid und |
| | 55 - 89 Mol-% Alkalisalz von Acrylsäure |
| (c) | 0,01 - 0,3 Mol, |
wobei (a) plus (b) 100 Mol-% ergibt und die Menge von (c) als Mol pro 100 Mol von (a) und (b) angegeben ist.

15. Polymeres nach Anspruch 14, wobei es sich beim Sulfonat-Anion um 2-Acrylamido-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 1,5 - 15 Mol-% |
| (b) | 10 - 21 Mol-% Acrylamid und |
| | 64 - 88,5 Mol-% Alkalisalz von Acrylsäure |
| (c) | 0,01 - 0,2 Mol |
wobei (a) plus (b) 100 Mol-% ergibt und die Menge von (c) als Mol pro 100 Mol von (a) und (b) angegeben ist.

16. Polymeres nach Anspruch 5, wobei es sich beim Ammonium-Kation um 2-Methacryloyloxyethyldimethylammonium handelt.

17. Polymeres nach Anspruch 16, wobei es sich beim Comonomeren um Acrylamid handelt und das Polymere durch Polymerisation der folgenden Mengen der Komponenten (a), (b) und (c) gebildet worden ist:
| | **Bereich** |
|---|---|
| (a) | 3 - 50 Mol-% |
| (b) | 50 - 97 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

18. Polymeres nach Anspruch 17, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 3 - 20 Mol-% |
| (b) | 97 - 80 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

19. Polymeres nach Anspruch 17, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 10 - 50 Mol-% |
| (b) | 50 - 90 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

20. Polymeres nach Anspruch 16, wobei es sich beim Comonomeren um Acrylnitril handelt und das Polymere durch Polymerisation der folgenden Mengen der Komponenten (a), (b) und (c) gebildet worden ist:
| | **Bereich** |
|---|---|
| (a) | 6 - 25 Mol-% |
| (b) | 75 - 94 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

21. Polymeres nach Anspruch 20, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 10 - 20 Mol-% |
| (b) | 90 - 80 Mol-% |
| (c) | 0,01 - 0,1 Mol, |
wobei (a) plus (b) 100 Mol-% ergibt und die Menge von (c) als Mol pro 100 Mol von (a) und (b) angegeben ist.

22. Polymeres nach Anspruch 20, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 15 - 20 Mol-% |
| (b) | 80 - 85 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

23. Polymeres nach Anspruch 16, wobei es sich beim Comonomeren um ein Alkalisalz von Acrylsäure handelt und das Polymere durch Polymerisation der folgenden Mengen der Komponenten (a), (b) und (c) gebildet worden ist:
| | **Bereich** |
|---|---|
| (a) | 3 - 6 Mol-% |
| (b) | 94 - 97 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

24. Polymeres nach Anspruch 23, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | etwa 3 Mol-% |
| (b) | etwa 97 Mol-% |
| (c) | 0,01 - 0,1 Mol, |
wobei (a) plus (b) 100 Mol-% ergibt und die Menge von (c) als Mol pro 100 Mol von (a) und (b) angegeben ist.

25. Polymeres nach Anspruch 23, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | etwa 3 Mol-% |
| (b) | etwa 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

26. Polymeres nach Anspruch 16, wobei es sich beim Comonomeren um ein Alkalisalz von 2-Acrylamido-2-methylpropansulfonsäure handelt und das Polymere durch Polymerisation der folgenden Mengen der Komponenten (a), (b) und (c) gebildet worden ist:
| | **Bereich** |
|---|---|
| (a) | 10 - 25 Mol-% |
| (b) | 75 - 90 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

27. Polymeres nach Anspruch 26, wobei es sich beim Sulfonat-Anion um 2-Acrylamido-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 10 - 25 Mol-% |
| (b) | 75 - 90 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

28. Polymeres nach Anspruch 16, wobei es sich beim Comonomeren um eine Kombination aus Acrylamid und einem Alkalisalz von 2-Acrylamido-2-methylpropansulfonsäure handelt und das Polymere durch Polymerisation der folgenden Mengen der Komponenten (a), (b) und (c) gebildet worden ist:
| | **Bereich** |
|---|---|
| (a) | 1 - 10 Mol-% |
| (b) | 13 - 25 Mol-% Acrylamid und |
| | 65 - 86 Mol-% Alkalisalz von 2-Acrylamid-2-methylpropansulfonsäure |
| (c) | 0,01 - 0,2 Mol, |
wobei (a) plus (b) 100 Mol-% ergibt und die Menge von (c) als Mol pro 100 Mol von (a) und (b) angegeben ist.

29. Polymeres nach Anspruch 28, wobei es sich beim Sulfonat-Anion um 2-Acrylamid-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 1 - 10 Mol-% |
| (b) | 13 - 25 Mol-% Acrylamid und |
| | 65 - 86 Mol-% Alkalisalz von 2 -Acrylamid-2-methylpropansulfonsäure |
| (c) | 0,01 - 0,2 Mol, |
wobei (a) plus (b) 100 Mol-% ergibt und die Menge von (c) als Mol pro 100 Mol von (a) und (b) angegeben ist.

30. Polymeres nach Anspruch 16, wobei es sich beim Comonomeren um eine Kombination aus Acrylamid und einem Alkalisalz von Acrylsäure handelt und das Polymere durch Polymerisation der folgenden Mengen der Komponenten (a), (b) und (c) gebildet worden ist:
| | **Bereich** |
|---|---|
| (a) | 1 - 30 Mol-% |
| (b) | 10 - 50 Mol-% Acrylamid und |
| | 20 - 89 Mol-% Alkalisalz von Acrylsäure |
| (c) | 0,01 - 0,3 Mol, |
wobei (a) plus (b) 100 Mol-% ergibt und die Menge von (c) als Mol pro 100 Mol von (a) und (b) angegeben ist.

31. Polymeres nach Anspruch 30, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 1 - 6 Mol-% |
| (b) | 13 - 26 Mol-% Acrylamid und |
| | 68 - 86 Mol-% Alkalisalz von Acrylsäure |
| (c) | 0,01 - 0,2 Mol, |
wobei (a) plus (b) 100 Mol-% ergibt und die Menge von (c) als Mol pro 100 Mol von (a) und (b) angegeben ist.

32. Polymeres nach Anspruch 30, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 3 - 25 Mol-% |
| (b) | 13 - 47 Mol-% Acrylamid und |
| | 28 - 84 Mol-% Alkalisalz von Acrylsäure |
| (c) | 0,01 - 0,2 Mol, |
wobei (a) plus (b) 100 Mol-% ergibt und die Menge von (c) als Mol pro 100 Mol von (a) und (b) angegeben ist.

33. Polymeres nach Anspruch 5, wobei es sich beim Ammonium-Kation um 2 -Methacryloyloxyethyldiethylammonium handelt.

34. Polymeres nach Anspruch 33, wobei es sich beim Comonomeren um Acrylamid handelt und das Polymere durch Polymerisation der folgenden Mengen der Komponenten (a) , (b) und (c) gebildet worden ist:
| | **Bereich** | **Bevorzugter Bereich** |
|---|---|---|
| (a) | 3 - 50 Mol-% | 3 - 30 Mol-% |
| (b) | 50 - 97 Mol-% | 70 - 97 Mol-% |
| (c) | 0,01 - 0,3 Mol | 0,03 - 0,2 Mol, |
wobei (a) plus (b) 100 Mol-% ergibt und die Menge von (c) als Mol pro 100 Mol von (a) und (b) angegeben ist.

35. Polymeres nach Anspruch 34, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 3 - 30 Mol-% |
| (b) | 70 - 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

36. Polymeres nach Anspruch 34, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 3 - 50 Mol-% |
| (b) | 50 - 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

37. Polymeres nach Anspruch 33, wobei es sich beim Comonomeren um Acrylnitril handelt und das Polymere durch Polymerisation der folgenden Mengen der Komponenten (a), (b) und (c) gebildet worden ist:
| | **Bereich** |
|---|---|
| (a) | 3 - 30 Mol-% |
| (b) | 70 - 97 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

38. Polymeres nach Anspruch 37, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 3 - 25 Mol-% |
| (b) | 75 - 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

39. Polymeres nach Anspruch 37, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 3 - 30 Mol-% |
| (b) | 70 - 97 Mol-% |
| (c) | 0,01 - 0,2 Mol, |
wobei (a) plus (b) 100 Mol-% ergibt und die Menge von (c) als Mol pro 100 Mol von (a) und (b) angegeben ist.

40. Polymeres nach Anspruch 33, wobei es sich beim Comonomeren um ein Alkalisalz von Acrylsäure handelt und das Polymere durch Polymerisation der folgenden Mengen der Komponenten (a), (b) und (c) gebildet worden ist:
| | **Bereich** |
|---|---|
| (a) | 3 - 6 Mol-% |
| (b) | 94 - 97 Mol-% |
| (c) | 0,01 - 0,3 Mol, |
wobei (a) plus (b) 100 Mol-% ergibt und die Menge von (c) als Mol pro 100 Mol von (a) und (b) angegeben ist.

41. Polymeres nach Anspruch 40, wobei es sich beim Sulfonat-Anion um 2-Acrylamido-2-methylpropansulfonat oder 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | etwa 3 Mol-% |
| (b) | etwa 97 Mol-% |
| (c) | 0,03 - 0,2 Mol, |
wobei (a) plus (b) 100 Mol-% ergibt und die Menge von (c) als Mol pro 100 Mol von (a) und (b) angegeben ist.

42. Polymeres nach Anspruch 33, wobei es sich beim Comonomeren um eine Kombination aus Acrylamid und einem Alkalisalz von Acrylsäure handelt und das Polymere durch Polymerisation der folgenden Mengen der Komponenten (a), (b) und (c) gebildet worden ist:
| | **Bereich** |
|---|---|
| (a) | 1 - 55 Mol-% |
| (b) | 10 - 55 Mol-% Acrylamid und |
| | 32 - 89 Mol-% Alkalisalz von Acrylsäure |
| (c) | 0,01 - 0,3 Mol, |
wobei (a) plus (b) 100 Mol-% ergibt und die Menge von (c) als Mol pro 100 Mol von (a) und (b) angegeben ist.

43. Polymeres nach Anspruch 42, wobei es sich beim Sulfonat-Anion um 2-Acrylamido-2-methylpropansulfonat handelt und das Polymere durch Polymerisation der folgenden Mengen der Komponenten (a), (b) und (c) gebildet worden ist:
| | **Bereich** |
|---|---|
| (a) | 3 - 15 Mol-% |
| (b) | 15 - 30 Mol-% Acrylamid und |
| | 55 - 82 Mol-% Alkalisalz von Acrylsäure |
| (c) | 0,01 - 0,2 Mol, |
wobei (a) plus (b) 100 Mol-% ergibt und die Menge von (c) als Mol pro 100 Mol von (a) und (b) angegeben ist.

44. Polymeres nach Anspruch 42, wobei es sich beim Sulfonat-Anion um 2-Methacryloyloxyethansulfonat handelt und das Polymere durch Polymerisation der folgenden Mengen der Komponenten (a), (b) und (c) gebildet worden ist:
| | **Bereich** |
|---|---|
| (a) | 1 - 55 Mol-% |
| (b) | 10 - 55 Mol-% Acrylamid und |
| | 32 - 89 Mol-% Alkalisalz von Acrylsäure |
| (c) | 0,01 - 0,2 Mol, |
wobei (a) plus (b) 100 Mol-% ergibt und die Menge von (c) als Mol pro 100 Mol von (a) und (b) angegeben ist.

45. Polymeres nach einem der Ansprüche 1 bis 4, wobei das Polymere aus den Komponenten (a), (b) und (c') gebildet worden ist.

46. Polymeres nach Anspruch 45, wobei während der Pfropfpolymerisation auch ein Vernetzungsmittel copolymerisiert wird.

47. Polymeres nach Anspruch 45, wobei die Komponente (c') ein Polysaccharid, bei dem es sich um eine lösliche Stärke, Cellulose, Glycogen oder eine beliebige Kombination von zwei oder mehr Bestandteilen davon handelt, oder Polypropylen ist, wobei insbesondere die Komponente (c') Polypropylen oder ein Polysaccharid, bei dem es sich um eine lösliche Stärke oder Cellulose handelt, ist; und wobei es sich beim Comonomeren um Acrylamid, Acrylnitril oder ein Alkalisalz von Acrylsäure handelt.

48. Polymeres nach Anspruch 47, wobei es sich beim Ammonium-Kation um 3-Methacrylamidopropyldimethylammonium (MPDMA) handelt und das Polymere 1 bis 50 Gew.-% der Komponente (c') und 50 bis 99 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | **Comonomeres** | | |
|---|---|---|---|
| **MPDMA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80, |
worin AM Acrylamid bedeutet, AN Acrylnitril bedeutet und X-AA ein Alkalisalz von Acrylsäure bedeutet.

49. Polymeres nach Anspruch 47, wobei es sich beim Ammonium-Kation um 3-Methacrylamidopropyldimethylammonium (MPDMA) handelt und das Polymere 5 bis 30 Gew.-% der Komponente (c') und 70 bis 95 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | | **Comonomeres** | | |
|---|---|---|---|---|
| **MPDMA/AMPS** | **MPDMA/MES** | **AM** | **AN** | **X-AA** |
| 5 - 25 | - | - | 75 - 95 | - |
| - | 5 - 20 | - | 80 - 95 | - |
| 5 - 20 | - | 95 - 80 | - | - |
| - | 10 - 20 | 90 - 80 | - | - |
| 3 - 15 | - | - | - | 97 - 85 |
| - | 3 - 10 | - | - | 97 - 90, |
worin AMPS 2-Acrylamido-2-methylpropansulfonat bedeutet, MES 2-Methacryloyloxyethansulfonat bedeutet, AM Acrylamid bedeutet, AN Acrylnitril bedeutet und X-AA ein Alkalisalz von Acrylsäure bedeutet.

50. Polymeres nach Anspruch 47, wobei es sich beim Ammonium-Kation um 3-Methacryloyloxyethyldimethylammonium (MEDMA) handelt und das Polymere 1 bis 50 Gew.-% der Komponente (c') und 50 bis 99 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | **Comonomeres** | | |
|---|---|---|---|
| **MEDMA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80, |
worin AM Acrylamid bedeutet, AN Acrylnitril bedeutet und X-AA ein Alkalisalz von Acrylsäure bedeutet.

51. Polymeres nach Anspruch 47, wobei es sich beim Ammonium-Kation um 2-Methacryloyloxyethyldimethylammonium (MEDMA) handelt und das Polymere 5 bis 30 Gew.-% der Komponente (c') und 70 bis 95 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | | **Comonomeres** | | |
|---|---|---|---|---|
| **MEDMA/AMPS** | **MEDMA/MES** | **AM** | **AN** | **X-AA** |
| 7 - 20 | - | - | 93 - 80 | - |
| - | 10 - 25 | - | 75 - 90 | - |
| 5 - 15 | - | 95 - 85 | - | - |
| - | 10 - 15 | 90 - 85 | - | - |
| 3 - 15 | - | - | | 97 - 85 |
| - | 3- 15 | - | | 97 - 85, |
worin AMPS 2-Acrylamido-2-methylpropansulfonat bedeutet, MES 2-Methacryloyloxyethansulfonat bedeutet, AM Acrylamid bedeutet, AN Acrylnitril bedeutet und X-AA ein Alkalisalz von Acrylsäure bedeutet.

52. Polymeres nach Anspruch 47, wobei es sich beim Ammonium-Kation um 3-Methacryloyloxyethyldiethylammonium (MEDEA) handelt und das Polymere 1 bis 50 Gew.-% der Komponente (c') und 50 bis 99 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | **Comonomeres** | | |
|---|---|---|---|
| **MEDEA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80, |
worin AM Acrylamid bedeutet, AN Acrylnitril bedeutet und X-AA ein Alkalisalz von Acrylsäure bedeutet.

53. Polymeres nach Anspruch 47, wobei es sich beim Ammonium-Kation um 3-Methacryloyloxyethyldiethylammonium (MEDEA) handelt und das Polymere 5 bis 30 Gew.-% der Komponente (c') und 70 bis 95 Gew.-% des gepfropften Comonomeren und ampholytischen Ionenpaars enthält, wobei die Mengen des Comonomeren und des ampholytischen Ionenpaars innerhalb der folgenden, in Mol-% angegebenen Bereiche liegen:
| **Ionenpaar** | | **Comonomeres** | | |
|---|---|---|---|---|
| **MEDEA/AMPS** | **MEDEA/MES** | **AM** | **AN** | **X-AA** |
| 5 - 20 | - | - | 95 - 80 | - |
| - | 5 - 20 | - | 95 - 80 | - |
| 5 - 15 | - | 95 - 85 | - | - |
| - | 10 - 15 | 90 - 85 | - | - |
| 3 - 15 | - | - | - | 97 - 85 |
| - | 3 - 10 | - | - | 97 - 90 |
worin AMPS 2-Acrylamido-2-methylpropansulfonat bedeutet, MES 2-Methacryloyloxyethansulfonat bedeutet, AM Acrylamid bedeutet, AN Acrylnitril bedeutet und X-AA ein Alkalisalz von Acrylsäure bedeutet.

54. Polymeres nach einem der vorstehenden Ansprüche, wobei das Polymere zumindest partiell hydrolysiert ist oder wobei das Polymere zumindest partiell neutralisiert ist.

55. Papierhandtuch, enthaltend das Polymere nach einem der vorstehenden Ansprüche.

56. Einmalwindel, enthaltend das Polymere nach einem der Ansprüche 1 bis 54.

57. Verfahren zum Absorbieren von wäßrigen Elektrolytlösungen, umfassend das Kontaktieren des Polymeren nach einem der Ansprüche 1 bis 54, des Papierhandtuchs nach Anspruch 55 oder der Einmalwindel nach Anspruch 56 mit einer wäßrigen Elektrolytlösung, wobei es sich bei der wäßrigen Elektrolytlösung insbesondere um Leitungswasser, Salzwasser, Sole oder Urin handelt.

58. Verfahren zur Herstellung eines Polymeren durch Copolymerisation der folgenden Komponenten:
(a) ein ampholytisches Ionenpaar-Monomeres, umfassend
(i) das Ammoniumkation 3-Methacrylamidopropyldimethylammonium, 2-Methacryloyloxyethyldimethylammonium oder 2 -Methacryloyloxyethyldiethylammonium und
(ii) ein Sulfonatanion, bei dem es sich um 2-Acrylamido-2-methylpropansulfonat, 2-Methacryloyloxyethansulfonat, Vinylsulfonat, Styrolsulfonat oder um eine Kombination aus zwei oder mehr dieser Bestandteile handelt; und
(b) mindestens ein Comonomeres, bei dem es sich handelt um Acrylamid, Methacrylamid, Acrylnitril, Acrylsäure, Methacrylsäure, ein Alkalisalz von Acrylsäure, ein Alkalisalz von Methacrylsäure, 2-Acrylamido-2-methylpropansulfonsäure, ein Alkalisalz von 2-Acrylamido-2-methylpropansulfonsäure, 2-Methacryloyloxyethansulfonsäure, ein Alkalisalz von 2-Methacryloyloxyethansulfonsäure, N-Vinyl-2-pyrrolidon oder ein Amin entsprechend dem Ammoniumkation (a) (i), nämlich 3-Methacrylamidopropyldimethylamin, 2-Methacryloyloxyethyldimethylamin bzw. 2-Methacryloyloxyethyldiethylamin oder eine Kombination von zwei oder mehr dieser Comonomeren;
und wobei ferner enthalten ist:
(c) ein Vernetzungsmittel, das mindestens zwei polymerisierbare olefinische Funktionalitäten aufweist, wobei sich die einzelnen olefinischen Funktionalitäten zur Vernetzung mit aus den Komponenten (a) und (b) gebildeten Polymerketten eignen, oder
(c') ein Polymeres, bei dem es sich um ein Polysaccharid, Polypropylen oder Polyethylen handelt, auf das das Comonomere (b) pfropfpolymerisiert ist und das ferner mit dem ampholytischen Ionenpaar-Monomeren (a) pfropfcopolymerisiert ist;
wobei die Comonomerkomponente (b) und die Ionenpaar-Monomerkomponente (a) in Mengen bereitgestellt werden, die zur Bildung eines stark absorbierenden Copolymeren befähigt sind.

## Revendications

1. Un polymère approprié pour être utilisé comme un absorbant d'une solution d'électrolyte aqueux, ledit polymère étant formé par la copolymérisation des composants suivants :
(a) un monomère à paire d'ions ampholytes comprenant :
(i) le cation d'ammonium qui est :
le 3-méthacrylamidopropyldiméthylammonium,
le 2-méthacryloyloxyéthyldiméthylammonium ou
le 2-méthacryloyloxyéthyldiéthylammonium et
(ii) un anion sulfonate qui est le sulfonate de 2-acrylamido-2-méthylpropane, le sulfonate de 2-méthacryloyloxyéthane, le sulfonate de vinyle, le sulfonate de styrène ou une combinaison de deux ou plusieurs de ceux-ci ; et
(b) au moins un comonomère qui est l'acrylamide, le méthacrylamide, l'acrylonitrile, l'acide acrylique, l'acide méthacrylique, un sel alcalin d'acide acrylique, un sel alcalin de l'acide méthacrylique, l'acide 2-acrylamido-2-méthylpropanesulfonique, un sel alcalin de l'acide 2-acrylamido-2-méthylpropane-sulfonique, l'acide 2-méthacryloyloxyéthanesulfonique, un sel alcalin de l'acide 2-méthacryloyloxyéthane-sulfonique, la N-vinyl-2-pyrrolidone ou une amine correspondant audit cation d'ammonium dans (a) à savoir la 3-méthacrylamidopropyldiméthylamine, la 2-méthacryloyloxyéthyldiméthylamine, ou la 2-méthacryloyloxyéthyldiéthylamine respectivement ou une combinaison de deux ou plusieurs desdits comonomères et où il est en outre incorporé :
(c) un agent de réticulation qui a au moins deux fonctionnalités oléfiniques polymérisables où chacune des fonctionnalités oléfiniques est appropriée pour la réticulation avec les chaînes de polymères formées à partir des composants (a) et (b) ou
(c') un polymère qui est un polysaccharide, le polypropylène ou le polyéthylène sur lequel le comonomère (b) est polymérisé par greffage et avec lequel il est, en outre, copolymérisé par greffage le monomère à paire d'ions ampholytes (a) ;
dans lequel le composant comonomère (b) et le composant monomère à paire d'ions (a) sont prévus selon des quantités qui sont efficaces pour produire un copolymère fortement absorbant.

2. Un polymère selon la revendication 1, dans lequel le composant d'agent de réticulation (c) est le N,N-diallylméthacrylamide, la diallylamine, l'acide N,N-bisacrylamidoacétique, l'ester de méthyle de l'acide N,N'-bisacrylamidoacétique, le N,N'-méthylènebis-acrylamide (méthylène-bis-acrylamide), le N,N-benzylidènebisacrylamide, l'acrylate d'allyle, le diisopropénylbenzène, le succinate de diallyle, le diacrylate d'éthylèneglycol, le diallylacrylamide, le divinylbenzène ou une combinaison de deux ou plusieurs de ceux-ci en particulier où l'agent de réticulation comprend le méthylène-bis-acrylamide.

3. Un polymère selon la revendication 1 ou 2, dans lequel le comonomère (b) est l'acrylamide, l'acrylonitrile, l'acide acrylique, un sel alcalin de l'acide acrylique, l'acide 2-acrylamido-2-méthylpropanesulfonique, un sel alcalin de l'acide 2-acrylamido-2-méthylpropanesulfonique ou n'importe quel combinaison de deux ou plusieurs desdits comonomères.

4. Un polymère selon l'une quelconque des revendications précédentes, dans lequel l'anion sulfonate est le sulfonate de 2-acrylamido-2-méthylpropane ou le sulfonate de 2-méthacryloyloxyéthane.

5. Un polymère selon l'une quelconque des revendications précédentes, dans lequel ledit polymère est formé à partir des composants (a), (b) et (c).

6. Un polymère selon la revendication 5, dans lequel ledit cation d'ammonium est le 3-méthacrylamidopropyldiméthylammonium.

7. Un polymère selon la revendication 6, dans lequel le comonomère est l'acrylamide et le polymère est formé par la polymérisation des quantités suivantes des composants (a), (b) et (c)
| | Gamme |
|---|---|
| (a) | 10 à 30 moles % |
| (b) | 70 à 90 moles % |
| (c) | 0,01 à 0,3 mole |
où (a) + (b) donne 100 moles % et la quantité de (c) est donnée sous la forme de moles pour 100 moles de (a) et (b).

8. Un polymère selon la revendication 7, dans lequel l'anion sulfonate est le sulfonate de 2-acrylamido-2-méthylpropane ou le sulfonate de 2-méthacryloyloxyéthane et le polymère est formé par la polymérisation des quantités suivantes des composants (a), (b) et (c)
| | Gamme |
|---|---|
| (a) | 10 à 30 moles % |
| (b) | 70 à 90 moles % |
| (c) | 0,01 à 0,2 mole |
où (a) + (b) donne 100 moles % et la quantité de (c) est donnée en moles pour 100 moles de (a) et (b).

9. Un polymère selon la revendication 6, dans lequel le comonomère est l'acrylonitrile et le polymère est formé par la polymérisation des quantités suivantes des composants (a), (b) et (c)
| | Gamme |
|---|---|
| (a) | 3 à 35 moles % |
| (b) | 65 à 97 moles % |
| (c) | 0,01 à 0,3 mole |
où (a) + (b) donne 100 moles % et la quantité de (c) est donnée en moles pour 100 moles de (a) et (b).

10. Un polymère selon la revendication 8 dans lequel l'anion sulfonate est le sulfonate de 2-acrylamido-2-méthylpropane ou le sulfonate de 2-méthacryloyloxyéthane et le polymère est formé par la polymérisation des quantités suivantes des composants (a), (b) et (c)
| | Gamme |
|---|---|
| (a) | 3 à 35 moles % |
| (b) | 65 à 97 moles % |
| (c) | 0,01 à 0,2 mole |
où (a) + (b) donne 100 moles % et la quantité de (c) est donnée en moles pour 100 moles de (a) et (b).

11. Un polymère selon la revendication 6, dans lequel le comonomère est un sel alcalin de l'acide acrylique et le polymère est formé par la polymérisation des quantités suivantes des composants (a), (b) et (c)
| | Gamme |
|---|---|
| (a) | 1 à 30 moles % |
| (b) | 70 à 99 moles % |
| (c) | 0,01 à 0,3 mole |
où (a) + (b) donne 100 moles % et la quantité de (c) est donnée en moles pour 100 moles de (a) et (b).

12. Un polymère selon la revendication 11, dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 1 à 15 moles % |
| (b) | 85 à 99 moles % |
| (c) | 0,01 à 0,2 mole |
où (a) + (b) donne 100 moles % et la quantité de (c) est donnée en moles pour 100 moles de (a) et (b).

13. Un polymère selon la revendication 11 dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 1 à 25 moles % |
| (b) | 75 à 99 moles % |
| (c) | 0,01 à 0,2 mole |
où (a) + (b) donne 100 moles % et la quantité de (c) est donnée en moles pour 100 moles de (a) et (b).

14. Un polymère selon la revendication 6, dans lequel le comonomère est une combinaison d'acrylamide et d'un sel alcalin de l'acide acrylique et le polymère est formé par la polymérisation des quantités suivantes des composants (a), (b) et (c)
| | Gamme |
|---|---|
| (a) | 1 à 20 moles % |
| (b) | 10 à 25 moles % d'acrylamide et |
| | 55 à 89 moles % du sel alcalin de l'acide acrylique |
| (c) | 0,01 à 0,3 mole |
où (a) + (b) donne 100 moles % et la quantité de (c) est donnée en moles pour 100 moles de (a) et (b).

15. Un polymère selon la revendication 14 dans lequel l'anion sulfonate est le sulfonate de 2-acrylamido-2-méthylpropane ou le sulfonate de 2-méthacryloyloxyéthane et le polymère est formé par la polymérisation des quantités suivantes des composants (a), (b) et (c)
| | Gamme |
|---|---|
| (a) | 1,5 à 15 moles % |
| (b) | 10 à 21 moles % d'acrylamide et |
| | 64 à 88,5 moles % du sel alcalin de l'acide acrylique |
| (c) | 0,01 à 0,2 mole |
où (a) + (b) donne 100 moles % et la quantité de (c) est donnée en moles pour 100 moles de (a) et (b).

16. Un polymère selon la revendication 5 dans lequel ledit cation d'ammonium est le 2-méthacryloyloxyéthyldiméthylammonium.

17. Un polymère selon la revendication 16, dans lequel le comonomère est l'acrylamide et le polymère est formé par la polymérisation des quantités suivantes des composants (a), (b) et (c)
| | Gamme |
|---|---|
| (a) | 3 à 50 moles % |
| (b) | 50 à 97 moles % |
| (c) | 0,01 à 0,3 mole |
où (a) + (b) donne 100 moles % et la quantité de (c) est donnée en moles pour 100 moles de (a) et (b).

18. Un polymère selon la revendication 17 dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 3 à 20 moles % |
| (b) | 97 à 80 moles % |
| (c) | 0,01 à 0,2 mole |
où (a) + (b) donne 100 moles % et la quantité de (c) est donnée en moles pour 100 moles de (a) et (b).

19. Un polymère selon la revendication 17 dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 10 à 50 moles % |
| (b) | 50 a 90 moles % |
| (c) | 0,01 à 0,2 mole |
où (a) + (b) donne 100 moles % et la quantité de (c) est donnée en moles pour 100 moles de (a) et (b).

20. Un polymère selon la revendication 16 dans lequel le comonomère est l'acrylonitrile et le polymère est formé par la polymérisation des quantités suivantes des composants (a), (b) et (c)
| | Gamme |
|---|---|
| (a) | 6 à 25 moles % |
| (b) | 75 à 94 moles % |
| (c) | 0,01 à 0,3 mole |
où (a) + (b) donne 100 moles % et la quantité de (c) est donnée en moles pour 100 moles de (a) et (b).

21. Un polymère selon la revendication 20, dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 10 à 20 moles % |
| (b) | 90 à 80 moles % |
| (c) | 0,01 à 0,1 mole |
où (a) + (b) donne 100 moles % et la quantité de (c) est donnée en moles pour 100 moles de (a) et (b).

22. Un polymère selon la revendication 20 dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 15 à 20 moles % |
| (b) | 80 à 85 moles % |
| (c) | 0,01 à 0,2 mole |
où (a) + (b) donne 100 moles % et la quantité de (c) est donnée en moles pour 100 moles de (a) et (b).

23. Un polymère selon la revendication 16, dans lequel le comonomère est un sel alcalin de l'acide acrylique et le polymère est formé par la polymérisation des quantités suivantes des composants (a), (b) et (c)
| | Gamme |
|---|---|
| (a) | 3 à 6 moles % |
| (b) | 94 à 97 moles % |
| (c) | 0,01 à 0,3 mole |
où (a) + (b) donne 100 moles % et la quantité de (c) est donnée en moles pour 100 moles de (a) et (b).

24. Un polymère selon la revendication 23 dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | environ 3 moles % |
| (b) | environ 97 moles % |
| (c) | 0,01 à 0,1 mole |
où (a) + (b) donne 100 moles % et la quantité de (c) est donnée en moles pour 100 moles de (a) et (b).

25. Un polymère selon la revendication 23 dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | environ 3 moles % |
| (b) | environ 97 moles % |
| (c) | 0,01 à 0,2 mole |
où (a) + (b) donne 100 moles % et la quantité de (c) est donnée en moles pour 100 moles de (a) et (b).

26. Un polymère selon la revendication 16, dans lequel le comonomère est un sel alcalin de l'acide 2-acrylamido-2-méthylpropanesulfonique et le polymère est formé par la polymérisation des quantités suivantes des composants (a), (b) et (c)
| | Gamme |
|---|---|
| (a) | 10 à 25 moles % |
| (b) | 75 à 90 moles % |
| (c) | 0,01 à 0,3 mole |
où (a) + (b) donne 100 moles % et la quantité de (c) est donnée en moles pour 100 moles de (a) et (b).

27. Un polymère selon la revendication 26, dans lequel l'anion sulfonate est le sulfonate de 2-acrylamido-2-méthylpropane ou le sulfonate de 2-méthacryloyloxyéthane et le polymère est formé par la polymérisation des quantités suivantes des composants (a), (b) et (c)
| | Gamme |
|---|---|
| (a) | 10 à 25 moles % |
| (b) | 75 à 90 moles % |
| (c) | 0,01 à 0,2 mole |
où (a) + (b) donne 100 moles % et la quantité de (c) est donnée en moles pour 100 moles de (a) et (b).

28. Un polymère selon la revendication 16 dans lequel le comonomère est une combinaison d'acrylamide et d'un sel alcalin de l'acide 2-acrylamido-2-méthylpropanesulfonique et le polymère est formé par la polymérisation des quantités suivantes des composants (a), (b) et (c)
| | Gamme |
|---|---|
| (a) | 1 à 10 moles % |
| (b) | 13 à 25 moles % d'acrylamide et |
| | 65 à 86 moles % du sel alcalin de l'acide 2-acrylamide-2-méthylpropanesulfonique |
| (c) | 0,01 à 0,2 mole |
où (a) + (b) donne 100 moles % et la quantité de (c) est donnée en moles pour 100 moles de (a) et (b).

29. Un polymère selon la revendication 28 dans lequel l'anion sulfonate est le sulfonate de 2-acrylamide-2-méthylpropane ou le sulfonate de 2-méthacryloyloxyéthane et le polymère est formé par la polymérisation des quantités suivantes des composants (a), (b) et (c).
| | Gamme |
|---|---|
| (a) | 1 à 10 moles % |
| (b) | 13 à 25 moles % d'acrylamide et |
| | 65 à 86 moles % du sel alcalin de l'acide 2-acrylamide-2-méthylpropanesulfonique |
| (c) | 0,01 à 0,2 mole |
où (a) + (b) donne 100 moles % et la quantité de (c) est donnée en moles pour 100 moles de (a) et (b).

30. Un polymère selon la revendication 16, dans lequel le comonomère est une combinaison d'acrylamide et d'un sel alcalin de l'acide acrylique et le polymère est formé par la polymérisation des quantités suivantes des composants (a), (b) et (c)
| | Gamme |
|---|---|
| (a) | 1 à 30 moles % |
| (b) | 10 à 50 moles % d'acrylamide et |
| | 20 à 89 moles % d'un sel alcalin de l'acide acrylique |
| (c) | 0,01 à 0,3 mole |
où (a) + (b) donne 100 moles % et la quantité de (c) est donnée en moles pour 100 moles de (a) et (b).

31. Un polymère selon la revendication 30, dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 1 à 6 moles % |
| (b) | 13 à 26 moles % d'acrylamide et |
| | 68 à 86 moles % d'un sel alcalin de l'acide acrylique |
| (c) | 0,01 à 0,2 mole |
où (a) + (b) donne 100 moles % et la quantité de (c) est donnée en moles pour 100 moles de (a) et (b).

32. Un polymère selon la revendication 30, dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 3 à 25 moles % |
| (b) | 13 à 47 moles % d'acrylamide et |
| | 28 à 84 moles % d'un sel alcalin de l'acide acrylique |
| (c) | 0,01 à 0,2 mole |
où (a) + (b) donne 100 moles % et la quantité de (c) est donnée en moles pour 100 moles de (a) et (b).

33. Un polymère selon la revendication 5, dans lequel ledit cation d'ammonium est le 2-méthacryloyloxyéthyldiéthylammonium.

34. Un polymère selon la revendication 33, dans lequel le comonomère est l'acrylamide et le polymère est formé par la polymérisation des quantités suivantes des composants (a), (b) et (c)
| | Gamme | Gamme préférée |
|---|---|---|
| (a) | 3 à 50 moles % | 3 à 30 moles % |
| (b) | 50 à 97 moles % | 70 à 97 moles % |
| (c) | 0,01 à 0,3 mole | 0,03 à 0,2 mole |
où (a) + (b) donne 100 moles % et la quantité de (c) est donnée en moles pour 100 moles de (a) et (b).

35. Un polymère selon la revendication 34 dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 3 à 30 moles % |
| (b) | 70 à 97 moles % |
| (c) | 0,01 à 0,2 mole |
où (a) + (b) donne 100 moles % et la quantité de (c) est donnée en moles pour 100 moles de (a) et (b).

36. Un polymère selon la revendication 34, dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 3 à 50 moles % |
| (b) | 50 à 97 moles % |
| (c) | 0,01 à 0,2 mole |
où (a) + (b) donne 100 moles % et la quantité de (c) est donnée en moles pour 100 moles de (a) et (b).

37. Un polymère selon la revendication 33, dans lequel le comonomère est l'acrylonitrile et le polymère est formé par la polymérisation des quantités suivantes des composants (a), (b) et (c)
| | Gamme |
|---|---|
| (a) | 3 à 30 moles % |
| (b) | 70 à 97 moles % |
| (c) | 0,01 à 0,3 mole |
où (a) + (b) donne 100 moles % et la quantité de (c) est donnée en moles pour 100 moles de (a) et (b).

38. Un polymère selon la revendication 37 dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 3 à 25 moles % |
| (b) | 75 à 97 moles % |
| (c) | 0,01 à 0,2 mole |
où (a) + (b) donne 100 moles % et la quantité de (c) est donnée en moles pour 100 moles de (a) et (b).

39. Un polymère selon la revendication 37 dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 3 à 30 moles % |
| (b) | 70 à 97 moles % |
| (c) | 0,01 à 0,2 mole |
où (a) + (b) donne 100 moles % et la quantité de (c) est donnée en moles pour 100 moles de (a) et (b).

40. Un polymère selon la revendication 33, dans lequel le comonomère est un sel alcalin de l'acide acrylique et le polymère est formé par la polymérisation des quantités suivantes des composants (a), (b) et (c)
| | Gamme |
|---|---|
| (a) | 3 à 6 moles % |
| (b) | 94 à 97 moles % |
| (c) | 0,01 à 0,3 mole |
où (a) + (b) donne 100 moles % et la quantité de (c) est donnée en moles pour 100 moles de (a) et (b).

41. Un polymère selon la revendication 40, dans lequel l'anion sulfonate est le sulfonate de 2-acrylamido-2-méthylpropane ou le sulfonate de 2-méthacryloyloxyéthane et le polymère est formé par la polymérisation des quantités suivantes des composants (a), (b) et (c)
| | Gamme |
|---|---|
| (a) | environ 3 moles % |
| (b) | environ 97 moles % |
| (c) | 0,03 à 0,2 mole |
où (a) + (b) donne 100 moles % et la quantité de (c) est donnée en moles pour 100 moles de (a) et (b).

42. Un polymère selon la revendication 33, dans lequel le comonomère est une combinaison d'acrylamide et d'un sel alcalin de l'acide acrylique et le polymère est formé par la polymérisation des quantités suivantes des composants (a), (b) et (c)
| | Gamme |
|---|---|
| (a) | 1 à 55 moles % |
| (b) | 10 à 55 moles % d'acrylamide et |
| | 32 à 89 moles % du sel alcalin de l'acide acrylique |
| (c) | 0,01 à 0,3 mole |
où (a) + (b) donne 100 moles % et la quantité de (c) est donnée en moles pour 100 moles de (a) et (b).

43. Un polymère selon la revendication 42, dans lequel l'anion sulfonate est le sulfonate de 2-acrylamido-2-méthylpropane et le polymère est formé par la polymérisation des quantités suivantes des composants (a), (b) et (c)
| | Gamme |
|---|---|
| (a) | 3 à 15 moles % |
| (b) | 15 à 30 moles % d'acrylamide et |
| | 55 à 82 moles % du sel alcalin de l'acide acrylique |
| (c) | 0,01 à 0,2 mole |
où (a) + (b) donne 100 moles % et la quantité de (c) est donnée en moles pour 100 moles de (a) et (b).

44. Un polymère selon la revendication 42, dans lequel l'anion sulfonate est le sulfonate de 2-méthacryloyloxyéthane et le polymère est formé par la polymérisation des quantités suivantes des composants (a), (b) et (c)
| | Gamme |
|---|---|
| (a) | 1 à 55 moles % |
| (b) | 10 à 55 moles % d'acrylamide et |
| | 32 à 89 moles % du sel alcalin de l'acide acrylique |
| (c) | 0,01 à 0,2 mole |
où (a) + (b) donne 100 moles % et la quantité de (c) est donnée en moles pour 100 moles de (a) et (b).

45. Un polymère selon l'une quelconque des revendications 1 à 4, dans lequel ledit polymère est formé à partir des composants (a), (b) et (c').

46. Un polymère selon la revendication 45, dans lequel pendant la polymérisation par greffage, il y a également un agent de réticulation copolymérisé.

47. Un polymère selon la revendication 45, dans lequel ledit composant (c') est un polysaccharide qui est un amidon, une cellulose, un glycogène soluble ou n'importe quelle combinaison de deux ou plusieurs de ceux-ci ou du polypropylène, en particulier dans lequel ledit composant (c') est du polypropylène ou un polysaccharide qui est un amidon ou une cellulose soluble et le comonomère est l'acrylamide, l'acrylonitrile ou un sel alcalin de l'acide acrylique.

48. Un polymère selon la revendication 47, dans lequel ledit cation d'ammonium est le 3-méthacrylamidopropyldiméthylammonium (MPDMA) et ledit polymère comprend 1 à 50 % en poids dudit composant (c') et 50 à 99 % en poids du comonomère et d'une paire d'ions ampholytes greffés avec, où les quantités du comonomère et de la paire d'ions ampholytes se situent dans l'une des gammes ci-après données en moles % :
| **Paire d'ions** | **Comonomère** | | |
|---|---|---|---|
| **MPDMA/Sulfonate** | **AM** | **AN** | **X-AA** |
| 2 - 25 | - | 98 - 75 | - |
| 2 - 20 | 98 -80 | - | - |
| 2 - 20 | - | - | 98 - 80 |
où AM est l'acrylamide, AN est l'acrylonitrile et X-AA est un sel alcalin de l'acide acrylique.

49. Un polymère selon la revendication 47, dans lequel ledit cation d'ammonium est le 3-méthacrylamidopropyldiméthylammonium (MPDMA) et ledit polymère comprend 5 à 30 % en poids du composant (c') et 70 à 95 % en poids du comonomère et de la paire d'ions ampholytes greffés avec, où les quantités du comonomère et de la paire d'ions ampholytes se situent dans l'une des gammes ci-après données en moles % :
| **Paire d'ions** | | **Comonomère** | | |
|---|---|---|---|---|
| **MPDMA/AMPS** | **MPDMA/MES** | **AM** | **AN** | **X-AA** |
| 5 - 25 | - | - | 75 - 95 | - |
| - | 5 - 20 | - | 80 - 95 | - |
| 5 - 20 | - | 95 - 80 | - | - |
| - | 10 - 20 | 90 - 80 | - | - |
| 3 - 15 | - | - | - | 97 - 85 |
| - | 3 - 10 | - | - | 97 - 90 |
où AMPS est le sulfonate de 2-acrylamido-2-méthylpropane, MES est le sulfonate de 2-méthacryloyloxyéthane, AM est l'acrylamide, AN est l'acrylonitrile et X-AA est un sel alcalin de l'acide acrylique.

50. Un polymère selon la revendication 47, dans lequel ledit cation d'ammonium est le 3-méthacryloyloxyéthyldiméthylammonium (MEDMA) et ledit polymère comprend 1 à 50 % en poids dudit composé (c') et 50 a 99 % en poids du comonomère et d'une paire d'ions ampholytes greffés avec, ou les quantités du comonomère et de la paire d'ions ampholytes se situent dans l'une des gammes ci-après données en moles % :
| **Paire d'ions** | **Comonomère** | | |
|---|---|---|---|
| **MEDMA/Sulfonate** | **AM** | **AN** | **X-AA** |
| 2 - 25 | - | 98 - 75 | - |
| 2 - 20 | 98 -80 | - | - |
| 2 - 20 | - | - | 98 - 80 |
ou AN est l'acrylamide, AN est l'acrylonitrile et X-AA est un sel alcalin de l'acide acrylique.

51. Un polymère selon la revendication 47, dans lequel ledit cation d'ammonium est le 2-méthacryloyloxyéthyldiméthylammonium (MEDMA) et ledit polymère comprend 5 à 30 % en poids du composant (c') et 70 à 95 % en poids du comonomère et de la paire d'ions ampholytes greffés avec, où les quantités du comonomère et de la paire d'ions ampholytes se situent dans l'une des gammes ci-après données en moles % :
| **Paire d'ions** | | **Comonomère** | | |
|---|---|---|---|---|
| **MEDMA/AMPS** | **MEDMA/MES** | **AM** | **AN** | **X-AA** |
| 7 - 20 | - | - | 93 - 80 | - |
| - | 10 - 25 | - | 70 - 90 | - |
| 5 - 15 | - | 95 - 85 | - | - |
| - | 10 - 15 | 90 - 85 | - | - |
| 3 - 15 | - | - | - | 97 - 85 |
| - | 3 - 15 | - | - | 97 - 85 |
où AMPS est le sulfonate de 2-acrylamido-2-méthylpropane, MES est le sulfonate de 2-méthacryloyloxyéthane, AM est l'acrylamide, AN est l'acrylonitrile et X-AA est un sel alcalin de l'acide acrylique.

52. Un polymère selon la revendication 47, dans lequel ledit cation d'ammonium est le 3-méthacryloyloxyéthyldiéthylammonium (MEDEA) et ledit polymère comprend 1 à 50 % en poids dudit composant (c') et 50 à 99 % en poids du comonomère et de la paire d'ions ampholytes greffés avec, où les quantités du comonomère et de la paire d'ions ampholytes se situent dans l'une des gammes suivantes données en moles %
| **Paire d'ions** | **Comonomère** | | |
|---|---|---|---|
| **MEDEA/Sulfonate** | **AM** | **AN** | **X-AA** |
| 2 - 25 | - | 98 - 75 | - |
| 2 - 20 | 98 - 80 | - | - |
| 2 - 20 | - | - | 98 - 80 |
où AM est l'acrylamide, AN est l'acrylonitrile et X-AA est un sel alcalin de l'acide acrylique.

53. Un polymère selon la revendication 47, dans lequel ledit cation d'ammonium est le 3-méthacryloyloxyéthyldiéthylammonium (MEDEA) et ledit polymère comprend 5 à 30 % en poids du composant (c') et 70 à 95 % en poids du comonomère et de la paire d'ions ampholytes greffés avec, où les quantités du comonomère et de la paire d'ions ampholytes se situent dans l'une des gammes ci-après données en moles % :
| **Paire d'ions** | | **Comonomère** | | |
|---|---|---|---|---|
| **MEDEA/AMPS** | **MEDEA/MES** | **AM** | **AN** | **X-AA** |
| 5 - 20 | - | - | 95 - 80 | - |
| - | 5 - 20 | - | 95 - 80 | - |
| 5 - 15 | - | 95 - 85 | - | - |
| - | 10 - 15 | 90 - 85 | - | - |
| 3 - 15 | - | - | - | 97 - 85 |
| - | 3 - 10 | - | - | 97 - 90 |
où AMPS est le sulfonate de 2-acrylamido-2-méthylpropane, MES est le sulfonate de 2-méthacryloyloxyéthane, AM est l'acrylamide, AN est l'acrylonitrile et X-AA est un sel alcalin de l'acide acrylique.

54. Un polymère selon l'une quelconque des revendications précédentes, dans lequel le polymère est au moins partiellement hydrolysé ou dans lequel le polymère est au moins partiellement neutralisé.

55. Une serviette en papier renfermant le polymère selon l'une quelconque des revendications précédentes.

56. Une couche à jeter après usage renfermant le polymère selon l'une quelconque des revendications 1 à 54.

57. Un procédé d'absorption de solutions d'électrolyte aqueux comprenant la mise en contact du polymère selon l'une quelconque des revendications 1 à 54, de la serviette en papier selon la revendication 55 ou de la couche à jeter après usage selon la revendication 56 avec une solution d'électrolyte aqueux en particulier dans lequel la solution d'électrolyte aqueux est de l'eau de ville, de l'eau saline, une saumure ou de l'urine.

58. Un procédé pour produire un polymère par copolymérisation des composants suivants :
(a) un monomère à paire d'ions ampholytes comprenant :
(i) le cation d'ammonium qui est :
le 3-méthacrylamidopropyldiméthylammonium,
le 2-méthacryloyloxyéthyldiméthylammonium ou
le 2-méthacryloyloxyéthyldiéthylammonium et
(ii) un anion sulfonate qui est le sulfonate de 2-acrylamido-2-méthylpropane, le sulfonate de 2-méthacryloyloxyéthane, le sulfonate de vinyle, le sulfonate de styrène ou une combinaison de deux ou plusieurs de ceux-ci ; et
(b) au moins un comonomère qui est l'acrylamide, le méthacrylamide, l'acrylonitrile, l'acide acrylique, l'acide méthacrylique, un sel alcalin de l'acide acrylique, un sel alcalin de l'acide méthacrylique, l'acide 2-acrylamido-2-méthylpropane-sulfonique, un sel alcalin de l'acide 2-acrylamido-2-méthylpropane-sulfonique, l'acide 2-méthacryloyloxyéthane-sulfonique, un sel alcalin de l'acide 2-méthacryloyloxyéthane-sulfonique, la N-vinyl-2-pyrrolidone ou une amine correspondant audit cation d'ammonium dans (a), à savoir la 3-méthacrylamidopropyldiméthylamine, la 2-méthacryloyloxyéthyldiméthylamine, ou la 2-méthacryloyloxyéthyldiéthylamine respectivement ou une combinaison de deux ou plusieurs desdits comonomères et où il est en outre incorporé :
(c) un agent de réticulation qui a au moins deux fonctionnalités oléfiniques polymérisables où chacune des fonctionnalités oléfiniques est appropriée pour la réticulation avec des chaînes de polymères formées à partir des composants (a) et (b) ou
(c') un polymère qui est un polysaccharide, du polypropylène ou du polyéthylène sur lequel le comonomère (b) est polymérisé par greffage et avec lequel il y a, en outre, le monomère à paire d'ions ampholytes (a) copolymérisé par greffage ;
où le composant copolymère (b) et le composant monomère à paire d'ions (a) sont prévus selon des quantités qui sont efficaces pour produire un copolymère fortement absorbant.
